# EUROPEAN PATENT APPLICATION

(11) **EP 4 722 376 A2**
(43) Date of publication of application: **08.04.2026**
(21) Application number: 25214456.3
(22) Date of filing: 14.12.2020
(51) Int. Cl.: C12P 19/34

(54) **CHIMERIC TERMINAL DEOXYNUCLEOTIDYL TRANSFERASES FOR TEMPLATE-FREE ENZYMATIC SYNTHESIS OF POLYNUCLEOTIDES**

(30) Priority: 12.12.2019 EP 19215628
(62) Divisional of application: 20819606.3
(71) Applicant: DNA Script, 94270 Le Kremlin-Bicêtre (FR)
(72) Inventor: CHAMPION, Elise, 94270 Le Kremlin-Bicêtre (FR); SOSKINE, Mikhael, 94270 Le Kremlin-Bicêtre (FR); JAZIRI, Faten, 94270 Le Kremlin-Bicêtre (FR); MCHALE, Daniel, 94270 Le Kremlin-Bicêtre (FR)
(74) Representative: Plasseraud IP

(57) **Abstract**

The invention is directed to compositions and methods for enzymatic template-free synthesis of polynucleotides using terminal deoxynucleotidyl transferases that are chimeras derived from variants from different species.

## Description

### BACKGROUND

The use of highly purified inexpensive polynucleotides of predetermined sequences in a wide range of lengths has become central to a host of technologies, including genomic and diagnostic sequencing, multiplex nucleic acid amplification, therapeutic antibody development, synthetic biology, nucleic acid-based therapeutics, DNA origami, DNA-based data storage, and the like. Recently, interest has arisen in supplementing or replacing chemically-based synthesis methods by enzymatically-based methods using template-free polymerases, such as, terminal deoxynucleotidyl transferase (TdT), because of the proven efficiency of such enzymes and the benefit of mild non-toxic reaction conditions, e.g. Ybert et al, International patent publication WO2015/159023; Hiatt et al, U.S. patent 5763594; Jensen et al, Biochemistry, 57: 1821-1832 (2018); and the like. Most approaches in enzyme-based synthesis require the use of reversibly blocked nucleoside triphosphates in order to obtain a desired sequence in the polynucleotide product. Unfortunately, natural TdTs incorporate such modified nucleoside triphosphates with reduced efficiency as compared to unmodified nucleoside triphosphates. Thus, a great deal of work has been directed to developing new TdT variants with better incorporation efficiencies for modified nucleoside triphosphates as well as other performance characteristics, such as, reduced incorporation variability among different 3'-sequences of a growing chain, improved manufacturability and the like, e.g. Champion et al, U.S. patent publication US2019/0211315; Ybert et al, International patent publication WO2017/216472, and the like.

In view of the above, the field of template-free enzymatically-based polynucleotide synthesis would be advanced if new template-free polymerases, such as new TdT variants, and methods of use, were available for improved stability and incorporation of reversibly blocked nucleoside triphosphates.

### SUMMARY OF THE INVENTION

The present invention is directed to methods and compositions for template-free enzymatic synthesis of polynucleotides. The invention includes deoxynucleotidyl transferase (TdT) variants, and compositions thereof, which are chimeras of TdT variants that comprise amino acid sequences derived from different species and that display one or more improvements, including improved efficiency in incorporating reversibly blocked nucleoside triphosphates into a polynucleotide, improved stability, improved manufacturability, or reduced variation in sequence-specific incorporation rates. The invention also includes methods and kits using such chimeric TdT variants for synthesizing polynucleotides of a predetermined sequence.

More particularly, the present invention relates to a chimeric terminal deoxynucleotidyl transferase (TdT) variant comprising an amino acid sequence at least 80 percent identical to the amino acid sequence defined by the formula:

B₁-B₂-B₃

wherein:
B₁ is an amino acid segment of a first species TdT without a BRCT-like region, extending from its N-terminus to an amino acid position between its VSC motif and its KMT motif inclusive, wherein B₁ comprises a substitution of methionine or functionally equivalent residue in its FMR motif, a substitution of the second arginine or functionally equivalent residue in its GGFRR motif whenever B₁ includes a GGFRR motif, and one or more substitutions of valine or serine or cysteine, or functionally equivalent residue, respectively, in its VSC motif whenever B₁ includes a VSC motif;
B₂ is an amino acid segment of a second species TdT extending from a C-terminus of B₁ to a C-terminus of the second species TdT, or to the N-terminus of B₃ whenever B₃ comprises one or more amino acids, wherein B₂ comprises one or more substitutions of valine or serine or cysteine, or functionally equivalent residue, respectively, in its VSC motif whenever B₂ includes a VSC motif, a substitution of the second arginine or functionally equivalent residue in its GGFRR motif whenever B₂ includes a GGFRR motif, a substitution of arginine or functionally equivalent residue in its TGSR motif whenever B₂ includes a TGSR motif, and a substitution of glutamic acid or functionally equivalent residue in its FER motif whenever B₂ includes a FER motif; and
B₃ is an amino acid segment of a third species TdT comprising from 0 to 70 amino acids and extending from a C-terminus of the third species TdT to a C-terminal amino acid of B₂, wherein B₃ comprises a substitution of arginine or functionally equivalent residue in its TGSR motif whenever B₃ includes a TGSR motif, and a substitution of glutamic acid or functionally equivalent residue in its FER motif whenever B₃ includes a FER motif;
and wherein the chimeric TdT variant (i) is capable of synthesizing a nucleic acid fragment without a template and (ii) is capable of incorporating a 3'-O-blocked nucleoside triphosphate onto a nucleic acid fragment;
and wherein B₁, B₂ and B₃ are selected so that the chimeric TdT variant has from 350 to 410 amino acids.

The present invention also relates to a chimeric terminal deoxynucleotidyl transferase (TdT) variant comprising an amino acid sequence at least 80 percent identical to an amino acid sequence defined by the formula:

J₁-J₂-J₃

Wherein:
J₁ is an amino acid segment of a mouse TdT without a BRCT-like region extending from its N-terminus to an amino acid position between a VSC motif and a KMT motif, inclusive, wherein J₁ comprises a substitution of methionine or functionally equivalent residue in a FMR motif, a substitution of the second arginine or functionally equivalent residue in its GGFRR motif whenever J₁ includes a GGFRR motif, and one or more substitutions of valine or serine or cysteine, or functionally equivalent residue, respectively, in the VSC motif whenever J₁ includes a VSC motif;
J₂ is an amino acid segment of a non-mouse TdT extending from an amino acid position located between a VSC motif and a KMT motif to an amino acid position at a TGSR motif, wherein the amino acid segment comprises a substitution of the second arginine or functionally equivalent residue in its GGFRR motif whenever J₂ includes a GGFRR motif, and one or more substitutions of valine or serine or cysteine, or functionally equivalent residue, respectively, in its VSC motif whenever J₂ includes a VSC motif; and
J₃ is an amino acid segment of a mouse TdT extending from an amino acid position at a TGSR motif to its C-terminal amino acid, wherein J₃ comprises a substitution of arginine or functionally equivalent residue in its TGSR motif whenever J₃ includes a TGSR motif, and a substitution of glutamic acid or functionally equivalent residue in its FER motif whenever J₃ includes a FER motif;
and wherein the chimeric TdT variant (i) is capable of synthesizing a nucleic acid fragment without a template and (ii) is capable of incorporating a 3'-O-blocked nucleoside triphosphate onto a nucleic acid fragment;
and wherein J₁, J₂ and J₃ are selected so that the chimeric TdT variant has from 350 to 410 amino acids.

In some embodiments, chimeric TdT variants of the invention comprise three contiguous segments: a N-terminal segment without a BRCT-like fragment, an internal segment and a C-terminal segment, wherein the N-terminal segment and the C-terminal segment are each from a TdT of a first species and the internal segment is from a TdT of a second species. In some embodiments, the boundary between the N-terminal segment and the internal segment is at a VSC motif and the boundary between the internal segment and the C-terminal segment is at a TGSR motif. In other embodiments, the boundary between the N-terminal segment and the internal segment is at a KMT motif and the boundary between the internal segment and the C-terminal segment is at a TGSR motif. In other embodiments, the boundary between the N-terminal segment and the internal segment is at a VSC motif and the boundary between the internal segment and the C-terminal segment is at a KMT motif. As used herein, the occurrence of a boundary between segments at an indicated motif means that the boundary may be located in the motif or from 1 to 15 amino acids in an N-terminal direction from the motif's N-terminal amino acid or from 1 to 15 amino acids in a C-terminal direction from the motif's C-terminal amino acid. In some embodiments, such boundary locations may be from 1 to 10 amino acids in an N-terminal direction from the motif's N-terminal amino acid or from 1 to 10 amino acids in a C-terminal direction from the motif's C-terminal amino acid. In some embodiments, such boundary locations may be from 1 to 5 amino acids in an N-terminal direction from the motif's N-terminal amino acid or from 1 to 5 amino acids in a C-terminal direction from the motif's C-terminal amino acid. In some embodiments, the N-terminal segment and the C-terminal segment of the above chimeric TdT variants are each from a mouse TdT variant. In some embodiments, such mouse segments are subject to one or more mutations, or a plurality of mutations, as described in Table 2.

In some embodiments, the invention comprises isolated chimeric TdT variants described below.

In one aspect, the invention is directed to methods of using chimeric TdT variants of the invention to synthesize a polynucleotide comprising the steps of (a) providing an initiator having a free 3'-hydroxyl; and (b) repeating cycles of (i) contacting under elongation conditions the initiator or elongated fragments having free 3'-O-hydroxyls with a 3'-O-blocked nucleoside triphosphate and a chimeric TdT variant so that the initiator or elongated fragments are elongated by incorporation of a 3'-O-blocked (and optionally base protected) nucleoside triphosphate to form 3'-O-blocked elongated fragments, and (ii) deblocking the elongated fragments to form elongated fragments having free 3'-hydroxyls, until the polynucleotide is synthesized.

The present invention further relates to a kit for performing a nucleotide incorporation reaction comprising: a) a chimeric TdT variant according to any one of claims 1 to 20, b) one or more nucleotides, preferably one or more 3'-O-blocked nucleosides triphosphates, and c) optionally at least one nucleic acid primer.

### BRIEF DESCRIPTION OF THE DRAWINGS

Fig. 1 illustrates diagrammatically the steps of a method of template-free enzymatic nucleic acid synthesis using TdT variants of the invention.
Fig. 2 illustrates diagrammatically the structure of chimeric TdT variants of the invention.
Figs. 3A-3B illustrate data showing percentage of deletions occurring at different 3-mer sequences (or "codons") during synthesis with TdT variants M27, M57 and M59.

### DETAILED DESCRIPTION OF THE INVENTION

While the invention is amenable to various modifications and alternative forms, specifics thereof have been shown by way of example in the drawings and will be described in detail. It should be understood that the intention is not to limit the invention to the particular embodiments described. It is the intention to cover all modifications, equivalents, and alternatives falling within the spirit and scope of the invention. Guidance for aspects of the invention is found in many available references and treatises well known to those with ordinary skill in the art.

The invention is directed to chimeric TdT variants and their use in template-free, or template-independent, synthesis of polynucleotides of a given (or equivalently, predetermined, or user-determined) sequence using 3'-O-reversibly blocked nucleoside triphosphates. The TdT variants of the invention are "chimeric" in the sense that they comprise a plurality of amino acid segments from two or more different biological species, usually different mammalian species. In some embodiments, the plurality of amino acid segments is 2 or 3, and in some embodiments, such segments may be selected from 2 different species and, in other embodiments, 3 different species. That is, in some embodiments, 2 or 3 segments are taken from 2 different species. Whenever 3 segments are from 2 different species, two segments from a first species are at the N-terminus and C-terminus of the resulting chimeric TdT variant and a second species TdT segment is sandwiched in between them. In each case, the selected segments are from TdT variants that have amino acid substitutions at positions indicated below that enhance the incorporation activity of the chimeric TdT variants for 3'-O-reversibly blocked nucleoside triphosphates. In some embodiments, positions of boundaries between segments from the different species and the positions of amino acid substitutions in chimeric TdT variants of the invention are defined in relation to multiple sequence motifs that occur in the amino acid sequences of all TdT proteins, especially mammalian TdT proteins and that are well-known in the scientific literature or defined herein. The ordering of the amino acid segments in chimeric TdT variants of the invention preserves the ordering and numbers of sequence motifs found in natural TdTs. That is, each natural TdT has an ordering and number of sequence motifs (e.g. from N-terminal to C-terminal: FMR, VSC, GGFRR, KMT, TGSR, FER, etc.). Each chimeric TdT variant of the invention has the same ordering and number of such sequence motifs.

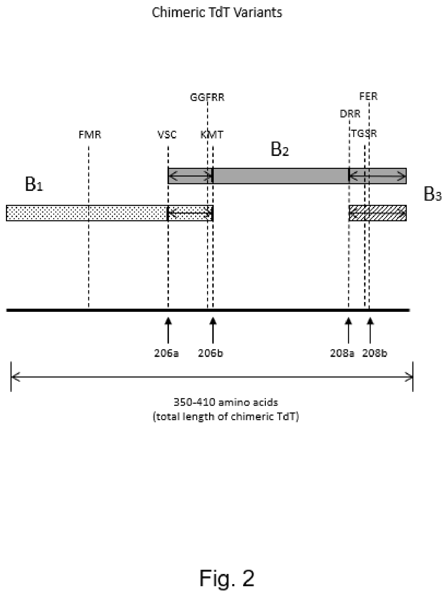

As illustrated in Fig. 2 for some embodiments of a chimeric TdT variant of the invention comprising three segments from three different species: B₁ from species 1 TdT variant (200), B₂ from species 2 TdT variant (202), and B₃ from species 3 TdT variant (204). Chimeric TdTs of the invention do not include an N-terminal BRCT-like segment typically comprising about 130 amino acids in the wild type, or naturally occurring, TdTs, e.g. Delarue et al, EMBO J., 21(3): 427-439 (2002). That is, all chimeric TdT variants of the invention are N-terminally truncated by their BRCT-like segments. In some embodiments, the N-terminal amino acid of a B₁ segment is readily determined from a full length TdT by aligning its sequence with the truncated mouse TdT of SEQ ID NO: 2, or like sequence, using a conventional sequence alignment tool, e.g. Multalin (Mitchell, Bioinformatics, 9(5): 614-615

(1993)). As denoted by arrows (206a, 206b, 208a and 208b), the boundaries between segments B₁ and B₂ and between segments B₂ and B₃ may vary for different chimeric TdT variants within the indicated regions defined by the positions of the indicated sequence motifs (and shown by the arrows): the B₁-B₂ boundary may be located at any amino acid position between the VSC motif and the KMT motif and the B₂-B₃ boundary may be located at any amino acid position between a DRR motif (or equivalently the amino acid position about 70 amino acids from the C-terminus) and the C-terminus of the chimeric TdT variant. That is, in some embodiments, segment B₃ may contain zero amino acids or, in other words, may not be present so that the embodiment consists of only two segments from two different species.

Well-known TdT sequence motifs used herein include the GGFRR (or TGGFRRG) motif positioned in or near the 330-340 amino acid segment (measured in reference to the full length mouse TdT, e.g. SEQ ID NO: 1) and the TSGR motif positioned in or near the 450-460 amino acid segment (again, measured in reference to the full length mouse TdT, e.g. SEQ ID NO: 1). These and the additional sequence motifs FRM (positioned near the 185-195 amino acid segment with respect to SEQ ID NO: 1), VSC (positioned near the 295-305 amino acid segment with respect to SEQ ID NO: 1), KMT (positioned near the 335-345 amino acid segment with respect to SEQ ID NO: 1) and FER(positioned near the 450-465 amino acid segment with respect to SEQ ID NO: 1) are used to define some embodiments of the present invention. Alternatively, amino acid substitutions in the KMT motif may be equivalently defined as amino acid substitutions in the segment between the GGFRR motif and the GHD motif (which is positioned at or near the amino acid segment 34-345 with respect to SEQ ID NO: 1). In some embodiments, the KMT motif may alternatively be defined as the amino acid segment between the TGGFRRG motif and the GHD motif (non-inclusive), e.g. Delarue et al (cited above). In other embodiments, the KMT motif is a 3-amino acid segment between the TGGFRRG motif and the GHD motif selected from the group consisting of KMT, KKI, KKM, KEF, KKV, KKL and KKT. In some embodiments, the FMR motif is a 3-amino acid segment positioned in or near the 185-200 amino acid segment (re SEQ ID NO: 1) selected from the group consisting of FMR, FLR and YMR. In some embodiments, the VSC motif is a 3-amino acid segment positioned in or near the amino acid segment 295-305 (re SEQ ID NO: 1) selected from the group consisting of VSC, IDC, ISS, TSY, SKA, SKP, SRA and ASC. In some embodiments, the TGSR motif is a 4-amino acid segment positioned in or near the amino acid segment 445-460 (re SEQ ID NO: 1) selected from the group consisting of TGSR, TGSP and SGSR. In some embodiments, the FER motif is a 3-amino acid segment positioned in or near the amino acid segment 454-460 (re SEQ ID NO: 1) selected from the group consisting of FER and FGR. As used in reference to sequence motifs, in some embodiments, the term "near" or "at" means within 15 amino acids of the indicated range or motif, or within 10 amino acids of the indicated range or motif. The positions of the various motifs may vary from species to species of TdT, however, the ordering of the motifs along the TdT amino acid sequence is the same for all species and the relative spacings (in amino acids) between pairs of sequence motifs are highly conserved, as shown for selected motifs in the following Table 1. Thus, the motifs are useful milestones for defining amino acid segments making up chimeric TdTs of the invention.

**Table 1**

| Species | N-term | VSC | TGSR | Total | SEQ ID NO |
|---|---|---|---|---|---|
| | To VSC# | To TGSR* | To C-terminus^ | amino acids | |
| mouse | 170 | 155 | 56 | 381 | 2 |
| bovine | 170 | 154 | 56 | 380 | 3 |
| human | 170 | 154 | 56 | 380 | 4 |
| chicken | 169 | 151 | 56 | 376 | 5 |
| possum | 170 | 161 | 56 | 387 | 6 |
| shrew | 170 | 155 | 56 | 381 | 7 |
| dog | 170 | 155 | 56 | 381 | 8 |
| mole | 171 | 155 | 56 | 382 | 9 |
| pika | 168 | 155 | 56 | 379 | 10 |
| hedgehog | 170 | 158 | 56 | 384 | 11 |
| Tree shrew | 170 | 155 | 56 | 381 | 12 |
| platypus | 179 | 159 | 56 | 394 | 13 |
| Jerboa | 173 | 155 | 56 | 384 | 14 |
| puma | 153 | 151 | 56 | 364 | 15 |

| | | | | | |
|---|---|---|---|---|---|
| # does not include VSC * includes of VSC & TGSR ^ does not include TGSR | | | | | |

In some embodiments, a chimeric TdT variant of the invention has an amino acid sequence at least 80 percent identical to an amino acid sequence defined by the formula:

B₁-B₂-B₃

wherein:
B₁ is an amino acid segment of a first species TdT without a BRCT-like region, extending from its N-terminus to an amino acid position between its VSC motif and its KMT motif, wherein B₁ comprises a substitution of methionine or functionally equivalent residue in its FMR motif, a substitution of the second arginine (from N-terminus to C-terminus) or functionally equivalent residue in its GGFRR motif whenever B₁ includes a GGFRR motif, and one or more substitutions of valine or serine or cysteine, or functionally equivalent residue, respectively, in its VSC motif whenever B₁ includes a VSC motif;
B₂ is an amino acid segment of a second species TdT extending from a C-terminus of B₁ to a C-terminus of the second species TdT, or to the N-terminus of B₃ whenever B₃ comprises one or more amino acids, wherein B₂ comprises one or more substitutions of valine or serine or cysteine, or functionally equivalent residue, respectively, in its VSC motif whenever B2 includes a VSC motif, a substitution of the second arginine or functionally equivalent residue in its GGFRR motif whenever B₂ includes a GGFRR motif, a substitution of arginine or functionally equivalent residue in its TGSR motif whenever B₂ includes a TGSR motif, and a substitution of glutamic acid or functionally equivalent residue in its FER motif whenever B₂ includes a FER motif; and
B₃ is an amino acid segment of a third species TdT comprising from 0 to 70 amino acids and extending from a C-terminus of the third species TdT to a C-terminal amino acid of B₂, wherein B₃ comprises a substitution of arginine or functionally equivalent residue in its TGSR motif whenever B₃ includes a TGSR motif, and a substitution of glutamic acid or functionally equivalent residue in its FER motif whenever B₃ includes a FER motif; and wherein the chimeric TdT variant (i) is capable of synthesizing a nucleic acid fragment without a template and (ii) is capable of incorporating a 3'-O-blocked nucleoside triphosphate onto a nucleic acid fragment; and wherein B₁, B₂ and B₃ are selected so that the chimeric TdT variant has from 350 to 410 amino acids.

In some embodiments, a chimeric TdT variant described above have B₁, B₂ and B₃ segments selected from the amino acid sequences of the group consisting of SEQ ID NOs 2, 3, 4, 5, 6, 7, 8, 9, 10, 11, 12, 13, 14, 15, 27, 27, 28, 29, 30, 31, 32, 33, 34, 35, 36, 37, 38, 39, 40 and 41, wherein: each B₁ segment has (i) a substitution of a methionine or its functionally equivalent residue in the FMR motif is at position 63 with respect to SEQ ID NOs: 2, 3, 4, 6, 7, 8, 11, 12 and 13; at position 62 with respect to SEQ ID NO: 5; position 64 with respect to SEQ ID NO: 9; position 61 with respect to SEQ ID NO: 10, 26, 28, 29, 30, 32, 33, 34, 36, 37, 38 and 39; position 66 with respect to SEQ ID NO: 14; position 47 with respect to SEQ ID NO: 15; and position 48 with respect to SEQ ID NO: 27, 31, 35 and 40; (ii) a substitution of a valine or its functionally equivalent residue in the VSC motif is at position 171 with respect to SEQ ID NOs: 2, 3, 4, 6, 7, 8, 11 and 12; position 170 with respect to SEQ ID NO: 5, 32, 33, 34, 36, 37, 38, 39 and 41; position 172 with respect to SEQ ID NO: 9; position 169 with respect to SEQ ID NO: 10, 28, 29, 30, 32, 33, 34, 36, 37, 38, 38 and 41; position 180 with respect to SEQ ID NO: 13; position 174 with respect to SEQ ID NO: 14; position 154 with respect to SEQ ID NO: 15; position 168 with respect to SEQ ID NO: 26; position 156 with respect to SEQ ID NO: 27, 35 and 40; position 157 with respect to SEQ ID NO: 31; (iii) a substitution of a serine or its functionally equivalent residue in said VSC motif is at position 172 with respect to SEQ ID NOs: 2, 3, 4, 6, 7, 8, 11 and 12; position 171 with respect to SEQ ID NO: 5; position 173 with respect to SEQ ID NO: 9; position 170 with respect to SEQ ID NO: 10, 28, 29, 30; position 181 with respect to SEQ ID NO: 13; position 175 with respect to SEQ ID NO: 14; and position 155 with respect to SEQ ID NO: 15; position 169 with respect to SEQ ID NO: 26 and position 157 with respect to SEQ ID NO: 27, 31, 35 and 40; (iv) a substitution of a cysteine or its functionally equivalent residue in said VSC motif is at position 173 with respect to SEQ ID NOs: 2, 3, 4, 6, 7, 8, 11 and 12; position 172 with respect to SEQ ID NO: 5; position 174 with respect to SEQ ID NO: 9; position 171 with respect to SEQ ID NO: 10, 28, 29, 30, 32, 33, 34, 37, 38, 39 and 41; position 182 with respect to SEQ ID NO: 13; position 176 with respect to SEQ ID NO: 14; position 170 with respect to SEQ ID NO: 26; position 158 with respect to SEQ ID NO: 27, 31, 35, 36 and 40; and position 156 with respect to SEQ ID NO: 15; each B₂ segment has a substitution of a second arginine or its functionally equivalent residue in said GGFRR motif is at position 207 with respect to SEQ ID NOs: 2, 3, 4, 6, 7, 8, 11 and 12; position 206 with respect to SEQ ID NO: 5; position 208 with respect to SEQ ID NO: 9; position 205 with respect to SEQ ID NO: 10, 28, 29, 30, 32, 33, 34, 36, 37, 38, 39 and 41; position 204 with respect to SEQ ID NO: 26; position 192 with respect to SEQ ID NO: 27, 31, 35 and 40; position 216 with respect to SEQ ID NO: 13; position 210 with respect to SEQ ID NO: 14; and position 190 with respect to SEQ ID NO: 15; and each B₃ segment (or B₂ segment in some embodiments) has (i) a substitution of an arginine or functionally equivalent residue in said TGSR motif is at position 325 with respect to SEQ ID NOs: 2, 7, 8, 12 and 33; position 324 with respect to SEQ ID NOs: 3, 4, 29 and 32; position 320 with respect to SEQ ID NO: 5; position 331 with respect to SEQ ID NO: 6; position 326 with respect to SEQ ID NO: 9 and 26; position 327 with respect to SEQ ID NO: 28, 30; position 323 with respect to SEQ ID NO: 10; position 328 with respect to SEQ ID NO: 11 and 14; position 338 with respect to SEQ ID NO: 13; position 314 with respect to SEQ ID NO: 27, 31; position 310 with respect to SEQ ID NO: 35; position 311 with respect to SEQ ID NO: 31; position 321 with respect to SEQ ID NO: 32; position 322 with respect to SEQ ID NO: 33, 34; position 323 with respect to SEQ ID NO: 36, 37, 38, 39 and 41; position 309 with respect to SEQ ID NO: 40; and position 308 with respect to SEQ ID NO: 15; and (ii) a substitution of a glutamic acid or functionally equivalent residue in the FER motif is at position 328 with respect to SEQ ID NO: 2, 7, 8 and 12; position 327 with respect to SEQ ID NO: 3, 4 and 29; position 323 with respect to SEQ ID NO: 5; position 334 with respect to SEQ ID NO: 6; position 329 with respect to SEQ ID NO: 9 and 26; position 326 with respect to SEQ ID NO: 10, 36, 37, 38, 39 and 41; position 331 with respect to SEQ ID NO: 11 and 14; position 341 with respect to SEQ ID NO: 13; position 317 with respect to SEQ ID NO: 27; position 330 with respect to SEQ ID NO: 28 and 30; position 312 with respect to SEQ ID NO: 40; position 313 with respect to SEQ ID NO: 35; position 314 with respect to SEQ ID NO: 31; position 324 with respect to SEQ ID NO: 32; position 325 with respect to SEQ ID NO: 33 and 34; and position 311 with respect to SEQ ID NO: 15.

In some embodiments, chimeric TdT variants of the invention comprise: (i) a substitution of methionine or functionally equivalent residue in the FMR motif which is R or Q; (ii) a substitution of a second arginine or functionally equivalent residue in the GGFRR motif which is L or N; and (iii) a substitution of cysteine or functionally equivalent residue, in the VSC motif which is G, R, P, A, V, S, N, Q, or D, or in some embodiments G or R; (iv) a substitution of arginine or functionally equivalent residue in said TGSR motif which is P, N or A; and (v) a substitution of glutamic acid or functionally equivalent residue in said FER motif which is N, L, T, or S.

In some embodiments, the above percent identity value is at least 80 percent identity with the indicated B₁-B₂-B₃ amino acid sequence; in some embodiments, the above percent identity value is at least 90 percent identity with the indicated B₁-B₂-B₃ amino acid sequence; in some embodiments, the above percent identity value is at least 95 percent identity with the indicated B1-B2-B3 amino acid sequence; in some embodiments, the above percent identity value is at least 97 percent identity; in some embodiments, the above percent identity value is at least 98 percent identity; in some embodiments, the above percent identity value is at least 99 percent identity.

In some embodiments, chimeric TdT variants comprise chimeras of mouse and bovine TdT variants wherein B₁ and B₃ are derived from mouse TdT variants as described above and B₂ is derived from a bovine TdT variant as described above. In particular, chimeric TdT variants of the invention may comprise B₁, B₂ and B₃ segments obtained from mouse TdT variant M53 (SEQ ID NO: 21) and bovine TdT variant M54 (SEQ ID NO: 22). In some such chimeric TdT variants, B₁ and B₃ are derived from M53 and B₂ is derived from M54.

In some embodiments, chimeric terminal deoxynucleotidyl transferase (TdT) variants comprise three segments of amino acids: (a) an N-terminal segment of a mouse TdT (without its BRCT-like fragment) extending from the N-terminus to a position at the VSC motif, (b) a central, or internal, segment from a non-mouse TdT extending from a position at the VSC motif to about 5-15 amino acids in the N-terminal direction of the TGSR motif (or to a position at the TGSR motif), and (c) a C-terminal segment from a mouse TdT extending from the position 5-15 amino acids in the N-terminal direction from the TGSR motif to the C-terminus of the mouse TdT segment.

In some embodiments, a chimeric terminal deoxynucleotidyl transferase (TdT) variant comprises an amino acid sequence at least 80 percent identical to an amino acid sequence defined by the formula:

J₁-J₂-J₃

wherein:
J₁ is an amino acid segment of a mouse TdT without a BRCT-like region extending from its N-terminus to an amino acid position between a VSC motif and a KMT motif, inclusive, wherein J₁ comprises a substitution of methionine or functionally equivalent residue in a FMR motif, a substitution of the second arginine or functionally equivalent residue in its GGFRR motif whenever J₁ includes a GGFRR motif, and one or more substitutions of valine or serine or cysteine, or functionally equivalent residue, respectively, in the VSC motif whenever J₁ includes a VSC motif;
J₂ is an amino acid segment of a non-mouse TdT extending from an amino acid position located between a VSC motif and a KMT motif to an amino acid position at a TGSR motif, wherein the amino acid segment comprises a substitution of the second arginine or functionally equivalent residue in its GGFRR motif whenever J₂ includes a GGFRR motif, and one or more substitutions of valine or serine or cysteine, or functionally equivalent residue, respectively, in its VSC motif whenever J₂ includes a VSC motif; and
J₃ is an amino acid segment of a mouse TdT extending from an amino acid position at a TGSR motif to its C-terminal amino acid, wherein J₃ comprises a substitution of arginine or functionally equivalent residue in its TGSR motif whenever J₃ includes a TGSR motif, and a substitution of glutamic acid or functionally equivalent residue in its FER motif whenever J₃ includes a FER motif; and wherein the chimeric TdT variant (i) is capable of synthesizing a nucleic acid fragment without a template and (ii) is capable of incorporating a 3'-O-blocked nucleoside triphosphate onto a nucleic acid fragment. As above, J₁, J₂ and J₃ are selected so that the chimeric TdT variant has from 350 to 410 amino acids.

In some embodiments, such chimeric TdT variants comprise an amino acid sequence at least eighty percent identical to an amino acid sequence defined by the formula:

J₁-J₂-J₃

wherein:
J₁ is an amino acid segment having an amino acid sequence selected from SEQ ID NO: 42, 71, 73 or 82;
J₂ is an amino acid segment having an amino acid sequence selected from the group consisting of SEQ ID NOs 44, 45, 46, 47, 48, 49, 50, 51, 52, 53, 54, 55, 56, 57, 58, 59, 60, 61, 62, 63, 64, 65, 66, 67, 68, 69 or 70, each with a substitution of arginine at position 35; and
J₃ is an amino acid segment having an amino acid sequence of SEQ ID NO: 43 with a substitution of arginine at position 12 and glutamic acid at position 15 with respect to SEQ ID NO: 43, or an amino acid sequence of SEQ ID NO: 72; and wherein the chimeric TdT variant (i) is capable of synthesizing a nucleic acid fragment without a template and (ii) is capable of incorporating a 3'-O-blocked nucleoside triphosphate onto a nucleic acid fragment.

In some embodiments, J₁ is an amino acid segment having an amino acid sequence of SEQ ID NO: 82; J₂ is an amino acid segment having an amino acid sequence selected from the group consisting of SEQ ID NO: 52, 53, 54, 55, 56, 57, 58, 59, 62 and 63; and J₃ is an amino acid segment having an amino acid sequence of SEQ ID NO: 43, with a substitution of arginine at position 12 and glutamic acid at position 15 with respect to SEQ ID NO: 43, or an amino acid sequence of SEQ ID NO: 72.

In some embodiments, the amino acid sequence of SEQ ID NO: 42 further has a substitution of methionine at position 63; the amino acid sequences of SEQ ID NOs: 44, 45, 46, 47, 48, 49, 50, 51, 52, 53, 54, 55, 56, 57, 58, 59, 60, 61, 62, 63, 64, 65, 66, 67, 68, 69 and 70 further each have a substitution of cysteine at position 1; and the amino acid sequence of SEQ ID NO: 43 further has a substitution of arginine at position 38.

In further embodiments, the substitution of methionine at position 63 is R or Q, the substitution of arginine at position 35 is L or N, and the substitution of cysteine at positions 1 is G or R.

In some embodiments, J₂ is an amino acid segment having an amino acid sequence selected from a mammalian TdT, such as SEQ ID NO: 52, 53, 54, 55, 56, 57, 58, 59, 62, 63, 64, 65, 66, 67, 69 or 70. In some embodiments, J2 is an amino acid segment selected from a bovine TdT, such as SEQ ID NO: 58.

In further embodiments, the amino acid sequence of SEQ ID NO: 42 further has substitutions of alanine at position 17, leucine at position 52, glycine at position 57, methionine at position 63 and alanine at position 108.

In some embodiments, the above percent identity value is at least 80 percent identity with the indicated J₁-J₂-J₃ amino acid sequence; in some embodiments, the above percent identity value is at least 90 percent identity with the indicated J₁-J₂-J₃ amino acid sequence; in some embodiments, the above percent identity value is at least 95 percent identity with the indicated J₁-J₂-J₃ amino acid sequence; in some embodiments, the above percent identity value is at least 97 percent identity; in some embodiments, the above percent identity value is at least 98 percent identity; in some embodiments, the above percent identity value is at least 99 percent identity.

The invention of the above embodiments is based in part on the discovery by Applicants that the above chimeric TdT variants, J₁-J₂-J₃, have higher thermal stability than previous TdT variants, such as M57 (SEQ ID NO: 23), based on conventional thermal shift assays, as described below. In some embodiments, such increases in thermal stability are in the range of from 1-2°C.

In further embodiments, a chimeric TdT variant comprises an amino acid sequence at least ninety percent identical to an amino acid sequence defined by the formula:

R₁-R₂-R₃

wherein:
R₁ is an amino acid segment of a mouse TdT without a BRCT-like fragment extending from an N-terminus to a position at a VSC motif;
R₂ is an amino acid segment of a non-mouse TdT extending from the position at the VSC motif to a position aat a TGSR motif, wherein a second arginine of a GGFRR motif of R₂ is substituted; and
R₃ is an amino acid segment of a mouse TdT extending from the position at the TGSR motif to a C-terminus, wherein an arginine of the TGSR motif is substituted and a glutamic acid of an FER motif of R₃ is substituted; and wherein the chimeric TdT variant (i) is capable of synthesizing a nucleic acid fragment without a template and (ii) is capable of incorporating a 3'-O-blocked nucleoside triphosphate onto a nucleic acid fragment.

In some embodiments, the C-terminal amino acid of R₁ is the second amino acid of the VSC motif; the N-terminal amino acid of R₂ is the third amino acid of the VSC motif; the C-terminal amino acid of R₂ is in the region of from 5 to 10 amino acids in an N-terminal direction from a TGSR motif.

In some embodiments, the above percent identity value is at least 95 percent identity with the indicated R₁-R₂-R₃ amino acid sequence; in some embodiments, the above percent identity value is at least 97 percent identity; in some embodiments, the above percent identity value is at least 98 percent identity; in some embodiments, the above percent identity value is at least 99 percent identity.

In some embodiments, a chimeric TdT variant comprises at least one amino acid segment from a murine TdT and at least one amino acid sequence from a bovine TdT. Such chimeric TdT variants include TdTs comprising amino acid sequences SEQ ID NO: 74 (M57-N27-8-13c7 without His tag), SEQ ID NO: 75 (46-557 without His tag), SEQ ID NO: 76 (46-342 without His tag), SEQ ID NO: 77 (46-571 without His tag), SEQ ID NO: 78 (M92 without His tag), SEQ ID NO: 79 (M93 without His tag), SEQ ID NO: 80 (M94 without His tag) and SEQ ID NO: 81 (M88 without His tag). Applicants have discovered that the above chimeric TdT variants have higher thermal stability than previous TdT variants, such as M57 (SEQ ID NO: 23), based on conventional thermal shift assays, as described below. In some embodiments, such increases in thermal stability are in the range of from 1-2°C.

Chimeric TdT variants of the invention may comprise amino acid segments from a plurality of different species that are additionally subject to mutations of selected amino acids. For example, chimeric TdT variants constructed from the following species TdTs may be subject to one or more, or to a plurality of, mutations as described in the following Table:

**Table 2: Examples of chimeric TdT variants**

| **SEQ ID NO** | Animal | Substitutions | | | | |
|---|---|---|---|---|---|---|
| **1** | Mouse | M192R/Q | C302G/R | R336L/N | R454P/N/A/V | E457N/L/T/S/K |
| **2** | Mouse | M63R/Q | C173G/R | R207L/N | R325P/N/A/V | E328N/L/T/S/K |
| **3** | Bovine | M63R/Q | C173G/R | R207L/N | R324P/N/A/V | E327N/L/T/S/K |
| **4** | Human | M63R/Q | C173G/R | R207L/N | R324P/N/A/V | E327N/L/T/S/K |
| **5** | Chicken | --- | C172G/R | R206L/N | R320P/N/A/V | --- |
| **6** | Possum | M63R/Q | C173G/R | R207L/N | R331P/N/A/V | E334N/L/T/S/K |
| **7** | Shrew | M63R/Q | C173G/R | R207L/N | --- | E328N/L/T/S/K |
| **8** | Canine | M63R/Q | C173G/R | R207L/N | R325P/N/A/V | E328N/L/T/S/K |
| **9** | Mole | M64R/Q | C174G/R | R208L/N | --- | E329N/L/T/S/K |
| **10** | Pika | M61R/Q | C171G/R | R205L/N | R323P/N/A/V | E326N/L/T/S/K |
| **11** | Hedgehog | M63R/Q | C173G/R | R207L/N | R328P/N/A/V | E331N/L/T/S/K |
| **12** | Tree shrew | --- | C173G/R | R207L/N | R325P/N/A/V | E328N/L/T/S/K |
| **13** | Platypus | M63R/Q | C182G/R | R216L/N | R338P/N/A/V | E341N/L/T/S/K |
| **14** | Jerboa | M66R/Q | C176G/R | R210L/N | R328P/N/A/V | E331N/L/T/S/K |
| **15** | Puma | M47R/Q | C156G/R | R190L/N | R308P/N/A/V | E311N/L/T/S/K |
| **26** | Canary | --- | C170G/R | R204L/N | R326P/N/A/V | E329N/L/T/S/K |
| **27** | Neopelma | --- | C158G/R | R192L/N | R314P/N/A/V | E317N/L/T/S/K |
| **28** | Alligator | --- | --- | R205L/N | R327P/N/A/V | E330N/L/T/S/K |
| **29** | Xenopus | --- | --- | R205L/N | R324P/N/A/V | E327N/L/T/S/K |
| **30** | Tiger snake | --- | --- | R205L/N | R327P/N/A/V | E330N/L/T/S/K |
| **31** | Brown trout | --- | --- | R192L/N | R311P/N/A/V | E314N/L/T/S/K |
| **32** | Electric eel | --- | --- | R205L/N | R321P/N/A/V | E325N/L/T/S/K |
| **33** | Walking fish | --- | --- | R205L/N | R322P/N/A/V | E325N/L/T/S/K |
| **34** | Guppy | --- | --- | R205L/N | R322P/N/A/V | E325N/L/T/S/K |
| **35** | Rat | M48R/Q | C158G/R | R192L/N | R310P/N/A/V | E313N/L/T/S/K |
| **36** | Rat | M61R/Q | C171G/R | R205L/N | R323P/N/A/V | E326N/L/T/S/K |
| **37** | Colobus monkey | M61R/Q | C171G/R | R205L/N | R323P/N/A/V | E326N/L/T/S/K |
| **38** | Pig | M61R/Q | C171G/R | R205L/N | R323P/N/A/V | E326N/L/T/S/K |
| **39** | Tiger | M61R/Q | C171G/R | R205L/N | R323P/N/A/V | E326N/L/T/S/K |
| **40** | Water buffalo | M48R/Q | C158G/R | R192L/N | R310P/N/A/V | E313N/L/T/S/K |
| **41** | Marmot | M61R/Q | C171G/R | R205L/N | R323P/N/A/V | E326N/L/T/S/K |

Chimeric TdT variants of the invention may comprise amino acid segments having a percent sequence identity of a given segment in a specified SEQ ID NO, subject to the presence of indicated substitutions. In some embodiments, the number and type of sequence differences between a chimeric TdT variant of the invention described in this manner and the specified SEQ ID NO may be due to substitutions, deletion and/or insertions, and the amino acids substituted, deleted and/or inserted may comprise any amino acid. In some embodiments, such deletions, substitutions and/or insertions comprise only naturally occurring amino acids. In some embodiments, substitutions comprise only conservative, or synonymous, amino acid changes, as described in Grantham, Science, 185: 862-864 (1974). That is, a substitution of an amino acid can occur only among members of its set of synonymous amino acids. In some embodiments, sets of synonymous amino acids that may be employed are set forth in Table 3A.

**Table 3A: Synonymous Sets of Amino Acids I**

| Amino Acid | Synonymous Set |
|---|---|
| Ser | Ser, Thr, Gly, Asn |
| Arg | Arg, Gln, Lys, Glu, His |
| Leu | Ile, Phe, Tvr, Met, Val, Leu |
| Pro | Gly, Ala, Thr, Pro |
| Thr | Pro, Ser, Ala, Gly, His, Gln, Thr |
| Ala | Gly, Thr, Pro, Ala |
| Val | Met, Tyr, Phe, Ile, Leu, Val |
| Gly | Gly, Ala, Thr, Pro, Ser |
| Ile | Met, Tyr, Phe, Val, Leu, Ile |
| Phe | Trp, Met, Tyr, Ile, Val, Leu, Phe |
| Tyr | Trp, Met, Phe, Ile, Val, Leu, Tyr |
| Cys | Cys, Ser, Thr |
| His | His, Glu, Lvs, Gln, Thr, Arg |
| Gln | Gln, Glu, Lys, Asn, His, Thr, Arg |
| Asn | Asn, Gln, Asp, Ser |
| Lys | Lys, Glu, Gln, His, Arg |
| Asp | Asp, Glu, Asn |
| Glu | Glu, Asp, Lvs, Asn, Gln, His, Arg |
| Met | Met, Phe, Ile, Val, Leu |
| Trp | Trp |

In some embodiments, sets of synonymous amino acids that may be employed are set forth in Table 3B.

**Table 3B: Synonymous Sets of Amino Acids II**

| Amino Acid | Synonymous Set |
|---|---|
| Ser | Ser |
| Arg | Arg, Lys, His |
| Leu | Ile, Phe, Met, Leu |
| Pro | Ala, Pro |
| Thr | Thr |
| Ala | Pro, Ala |
| Val | Met, Ile Val |
| Gly | Gly |
| Ile | Met, Phe, Val, Leu, Ile |
| Phe | Met, Tyr, Ile, Leu, Phe |
| Tyr | Trp, Met |
| Cys | Cys, Ser |
| His | His, Gln, Arg |
| Gln | Gln, Glu, His |
| Asn | Asn, Asp |
| Lys | Lys, Arg |
| Asp | Asp, Asn |
| Glu | Glu, Gln |
| Met | Met, Phe, Ile, Val, Leu |
| Trp | Trp |

### Measurement of Nucleotide Incorporation Activity

The efficiency of nucleotide incorporation by variants of the invention may be measured by an extension, or elongation, assay, e.g. as described in Boule et al (cited below); Bentolila et al (cited below); and Hiatt et al, U.S. patent 5808045, the latter of which is incorporated herein by reference. Briefly, in one form of such an assay, a fluorescently labeled oligonucleotide having a free 3'-hydroxyl is reacted under TdT extension conditions with a variant TdT to be tested for a predetermined duration in the presence of a reversibly blocked nucleoside triphosphate, after which the extension reaction is stopped and the amounts of extension products and unextended initiator oligonucleotide are quantified after separation by gel electrophoresis. By such assays, the incorporation efficiency of a variant TdT may be readily compared to the efficiencies of other variants or to that of wild type or reference TdTs, or other polymerases. In some embodiments, a measure of variant TdT efficiency may be a ratio (given as a percentage) of amount of extended product using the variant TdT over the amount of extended product using wild type TdT in an equivalent assay.

In some embodiments, the following particular extension assay may be used to measure incorporation efficiencies of TdTs: Primer used is the following:
5'-AAAAAAAAAAAAAAGGGG-3' (SEQ ID NO: 20)
The primer has also an ATTO fluorescent dye on the 5' extremity. Representative modified nucleotides used (noted as dNTP in Table 4) include 3'-O-amino-2',3'-dideoxynucleotides-5'-triphosphates (ONH2, Firebird Biosciences), such as 3'-O-amino-2',3'-dideoxyadenosine-5'-triphosphate. For each different variant tested, one tube is used for the reaction. The reagents are added in the tube, starting from water, and then in the order of Table 4. After 30 min at 37°C the reaction is stopped by addition of formamide (Sigma).

**Table 4: Extension Activity Assay Reagents**

| Reagent | Concentration | Volume |
|---|---|---|
| H₂O | - | 12 µL |
| Activity buffer | 10x | 2 µL |
| dNTP | 250 µM | 2 µL |
| Purified enzyme | 20 µM | 2 µL |
| Fluorescent primer | 500 nM | 2 µL |

The Activity buffer comprises, for example, TdT reaction buffer (available from New England Biolabs) supplemented with CoCl₂.

The product of the assay is analyzed by conventional polyacrylamide gel electrophoresis. For example, products of the above assay may be analyzed in a 16 percent polyacrylamide denaturing gel (Bio-Rad). Gels are made just before the analysis by pouring polyacrylamide inside glass plates and let it polymerize. The gel inside the glass plates is mounted on an adapted tank filed with TBE buffer (Sigma) for the electrophoresis step. The samples to be analyzed are loaded on the top of the gel. A voltage of 500 to 2,000V is applied between the top and bottom of the gel for 3 to 6h at room temperature. After separation, gel fluorescence is scanned using, for example, a Typhoon scanner (GE Life Sciences). The gel image is analyzed using ImageJ software (imagej.nih.gov/ij/), or its equivalent, to calculate the percentage of incorporation of the modified nucleotides.

Hairpin completion assay. In one aspect, the invention includes methods of measuring the capability of a polymerase, such as a TdT variant, to incorporate a dNTP onto a 3' end of a polynucleotide (i.e. a "test polynucleotide"). One such method comprises providing a test polynucleotide with a free 3' hydroxyl under reaction conditions in which it is substantially only single stranded, but that upon extension with a polymerase, such as a TdT variant, forms a stable hairpin structure comprising a single stranded loop and a double stranded stem, thereby allowing detection of an extension of the 3' end by the presence of the double stranded polynucleotide. The double stranded structure may be detected in a variety of ways including, but not limited to, fluorescent dyes that preferentially fluoresce upon intercalation into the double stranded structure, fluorescent resonance energy transfer (FRET) between an acceptor (or donor) on the extended polynucleotide and a donor (or acceptor) on an oligonucleotide that forms a triplex with the newly formed hairpin stem, FRET acceptors and donors that are both attached to the test polynucleotide and that are brought into FRET proximity upon formation of a hairpin, or the like. In some embodiments, a stem portion of a test polynucleotide after extension by a single nucleotide is in the range of 4 to 6 basepairs in length; in other embodiments, such stem portion is 4 to 5 basepairs in length; and in still other embodiments, such stem portion is 4 basepairs in length. In some embodiments, a test polynucleotide has a length in the range of from 10 to 20 nucleotides; in other embodiments, a test polynucleotide has a length in the range of from 12 to 15 nucleotides. In some embodiments, it is advantageous or convenient to extend the test polynucleotide with a nucleotide that maximizes the difference between the melting temperatures of the stem without extension and the stem with extension; thus, in some embodiments, a test polynucleotide is extended with a dC or dG (and accordingly the test polynucleotide is selected to have an appropriate complementary nucleotide for stem formation).

Exemplary test polynucleotides for hairpin completion assays include p875 (5'-CAGTTAAAAACT) (SEQ ID NO: 16) which is completed by extending with a dGTP; p876 (5'- GAGTTAAAACT) (SEQ ID NO: 17) which is completed by extending with a dCTP; and p877 (5'- CAGCAAGGCT) (SEQ ID NO: 18) which is completed by extending with a dGTP. Exemplary reaction conditions for such test polynucleotides may comprise: 2.5 - 5 µM of test polynucleotide, 1:4000 dilution of GelRed^{®} (intercalating dye from Biotium, Inc., Fremont, CA), 200mM Cacodylate KOH pH 6.8, 1mM CoCl₂, 0-20% of DMSO and 3' ONH₂ dGTP and TdT at desired concentrations. Completion of the hairpin may be monitored by an increase in fluorescence of GelRed^{®} dye using a conventional fluorimeter, such as a TECAN reader at a reaction temperature of 28-38°C, using an excitation filter set to 360nm and anemission filter set to 635nm.

In some embodiments of this aspect of the invention, TdT variants may be tested for their capacity for template-free incorporate of nucleoside triphosphates by the following steps: (a) combining a test polynucleotide having a free 3'-hydroxyl, a TdT variant and a nucleoside triphosphate under conditions wherein the test polynucleotide is single stranded but upon incorporation of the nucleoside triphosphate forms a hairpin having a double stranded stem region, and (b) detecting the amount of double stranded stem regions formed as a measure of the capacity of the TdT variant to incorporate the nucleoside triphosphate. In some embodiments, the nucleoside triphosphate is a 3'-O-blocked nucleoside triphosphate.

### Measurement of TdT Variant Stability

Increases in the stability of a protein may be measured in a variety of ways. Fluorescent-based thermal shift assays are particularly useful for such measurements because of their simplicity and low cost. Exemplary references describing thermal shift assays and their application to measuring protein stability are as follows: Pantoliano et al, J. Biomolecular Screening, 6(6): 429-440 (2001); Huynh et al, Curr. Protocol. Protein Science, 79: 28.9.1-28.9.14; Ericsson et al, Anal. Biochem., 357: 289-298 (2006); Niesen et al, Nature Protocols, 2(9): 2212-2221 (2007); and Pantoliano et al, U.S. patent 6020141, the latter of which is hereby incorporated by reference. The conceptual basis for such assays is that folded and unfolded proteins can be distinguished through exposure to a hydrophobic fluorescent probe. Such a probe is quenched in aqueous solution but will preferentially bind to the exposed hydrophobic interior of an unfolding protein leading to a sharp decrease in quenching so that a readily detected fluorescence emission can be studied as a function of temperature. Thermally induced unfolding is an irreversible unfolding process following a typical two-state model with a sharp transition between the folded and unfolded states, where melting temperature (Tm) is defined as the midpoint of temperature of the protein-unfolding transition. Melting temperatures obtained with such a so-called "thermofluor" method have been shown to correlate well for several proteins with values determined by other biophysical methods used for measuring protein stability, such as circular dichroism (CD), turbidity measurements, and differential scanning calorimetry.

Low fluorescence at room temperature indicates a well-folded protein. Fluorescence emission increases with increasing temperature, giving rise to a sigmoidal curve that represents cooperative unfolding of the protein. The resulting sigmoidal curves may be fit to a Boltzmann Equation to identify the melting temperature that occurs at the midpoint of the unfolding transition; alternatively, the Tm is easily identified by plotting the first derivative of the fluorescence emission as a function of temperature (-dF/dT), where the Tm corresponds to the minimum of the curve, Huynh et al (cited above).

A variety of fluorescent dyes may be used and are commercially available directly or as part of an assay kit. An exemplary fluorescent dye is SYPRO Orange. A conventional real-time PCR instrument may be used for temperature control and fluorescent detection. Typically, a selection of buffers, salt concentrations and compositions, and other additives, e.g. DMSO, is made that corresponds to the incorporation or coupling conditions of a DNA synthesis reaction using a TdT variant. Exemplary coupling conditions are given below.

As mentioned above, in some embodiments, a stabilizing amino acid substitution (or equivalently, a stabilizing amino acid) at a specified position of an N-truncated TdT variant is an amino acid substitution which increases the melting temperature of said TdT variant by at least 1°C relative to a melting temperature of a TdT of the same amino acid sequence except for the stabilizing amino acid substitution, where the melting temperature is determined by a fluorescence-based thermal shift assay. In some embodiments, such assay uses a SYPRO Orange dye. In still further embodiments, reaction conditions of such assay comprise coupling reaction conditions. In further embodiments, such stabilizing amino acid substitution increases the melting temperature of a TdT variant by 1.5°C.

In some embodiments, melting temperature is measured with a thermal shift assay in the following solution: 0.5 Cacodylate KOH buffer pH 7.4 with SYPRO Orange dye. Some routine optimization of dye and protein concentrations may be made for the assay, e.g. guidance for such optimization is provided in the above-cited references, especially Huynh et al (cited above).

### Template-Free Enzymatic Synthesis

Template-free enzymatic synthesis of polynucleotides may be carried out by a variety of known protocols using template-free polymerases, such as terminal deoxynucleotidyl transferase (TdT), including variants thereof engineered to have improved characteristics, such as greater temperatue stability or greater efficiency in the incorporation of 3'-O-blocked deoxynucleoside triphosphates (3'-O-blocked dNTPs), e.g. Ybert et al, International patent publication WO/2015/159023; Ybert et al, International patent publication WO/2017/216472; Hyman, U.S. patent 5436143; Hiatt et al, U.S. patent 5763594; Jensen et al, Biochemistry, 57: 1821-1832 (2018); Mathews et al, Organic & Biomolecular Chemistry, DOI: 0.1039/c6ob01371f (2016); Schmitz et al, Organic Lett., 1(11): 1729-1731 (1999).

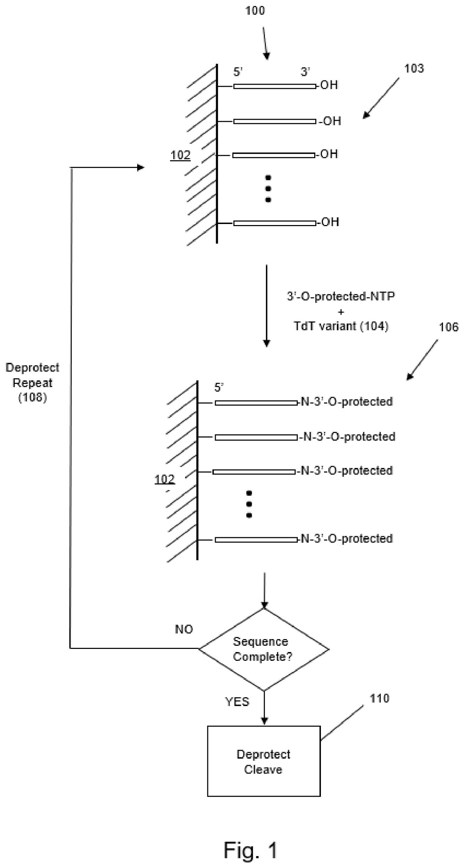

In some embodiments, the method of enzymatic DNA synthesis comprises repeated cycles of steps, such as are illustrated in Fig. 1, in which a predetermined nucleotide is added in each cycle. Initiator polynucleotides (100) are provided, for example, attached to solid support (102), which have free 3'-hydroxyl groups (103). To the initiator polynucleotides (100) (or elongated initiator polynucleotides in subsequent cycles) are added a 3'-O-protected-dNTP and a TdT variant under conditions (104) effective for the enzymatic incorporation of the 3'-O-protected-dNTP onto the 3' end of the initiator polynucleotides (100) (or elongated initiator polynucleotides). This reaction produces elongated initiator polynucleotides whose 3'-hydroxyls are protected (106). If the elongated initiator polynucleotide contains a competed sequence, then the 3'-O-protection group is removed, or deprotected, and the desired sequence is cleaved from the original initiator polynucleotide. Such cleavage may be carried out using any of a variety of single strand cleavage techniques, for example, by inserting a cleavable nucleotide at a predetermined location within the original initiator polynucleotide. An exemplary cleavable nucleotide may be a uracil nucleotide which is cleaved by uracil DNA glycosylase. If the elongated initiator polynucleotide does not contain a completed sequence, then the 3'-O-protection groups are removed to expose free 3'-hydroxyls (103) and the elongated initiator polynucleotides are subjected to another cycle of nucleotide addition and deprotection.

As used herein, the terms "protected" and "blocked" in reference to specified groups, such as, a 3'-hydroxyls of a nucleotide or a nucleoside, are used interchangeably and are intended to mean a moiety is attached covalently to the specified group that prevents a chemical change to the group during a chemical or enzymatic process. Whenever the specified group is a 3'-hydroxyl of a nucleoside triphosphate, or an extended fragment (or "extension intermediate") in which a 3'-protected (or blocked)-nucleoside triphosphate has been incorporated, the prevented chemical change is a further, or subsequent, extension of the extended fragment (or "extension intermediate") by an enzymatic coupling reaction.

In some embodiments, an ordered sequence of nucleotides is coupled to an initiator nucleic acid using a TdT in the presence of 3'-O-reversibly blocked dNTPs in each synthesis step. In some embodiments, the method of synthesizing an oligonucleotide comprises the steps of (a) providing an initiator having a free 3'-hydroxyl; (b) reacting under extension conditions the initiator or an extension intermediate having a free 3'-hydroxyl with a TdT in the presence of a 3'-O-blocked nucleoside triphosphate to produce a 3'-O-blocked extension intermediate; (c) deblocking the extension intermediate to produce an extension intermediate with a free 3'-hydroxyl; and (d) repeating steps (b) and (c) until the polynucleotide is synthesized. (Sometime "an extension intermediate" is also referred to as an "elongation fragment.") In some embodiments, an initiator is provided as an oligonucleotide attached to a solid support, e.g. by its 5' end. The above method may also include washing steps after the reaction, or extension, step, as well as after the de-blocking step. For example, the step of reacting may include a sub-step of removing unincorporated nucleoside triphosphates, e.g. by washing, after a predetermined incubation period, or reaction time. Such predetermined incubation periods or reaction times may be a few seconds, e.g. 30 sec, to several minutes, e.g. 30 min.

The above method may also include capping step(s) as well as washing steps after the reacting, or extending, step, as well as after the deblocking step. As mentioned above, in some embodiments, capping steps may be included in which non-extended free 3'-hydroxyls are reacted with compounds that prevents any further extensions of the capped strand. In some embodiments, such compound may be a dideoxynucleoside triphosphate. In other embodiments, non-extended strands with free 3'-hydroxyls may be degraded by treating them with a 3'-exonuclease activity, e.g. Exo I. For example, see Hyman, U.S. patent 5436143. Likewise, in some embodiments, strands that fail to be deblocked may be treated to either remove the strand or render it inert to further extensions.

In some embodiments that comprise serial synthesis of oligonucleotides, capping steps may be undesirable as capping may prevent the production of equal molar amounts of a plurality of oligonucleotides. Without capping, sequences will have a uniform distribution of deletion errors, but each of a plurality of oligonucleotides will be present in equal molar amounts. This would not be the case where non-extended fragments are capped.

In some embodiments, reaction conditions for an extension or elongation step may comprising the following: 2.0 µM purified TdT; 125-600 µM 3'-O-blocked dNTP (e.g. 3'-O-NH₂-blocked dNTP); about 10 to about 500 mM potassium cacodylate buffer (pH between 6.5 and 7.5) and from about 0.01 to about 10 mM of a divalent cation (e.g. CoCl₂ or MnCl₂), where the elongation reaction may be carried out in a 50 µL reaction volume, at a temperature within the range RT to 45°C, for 3 minutes. In embodiments, in which the 3'-O-blocked dNTPs are 3'-O-NH₂-blocked dNTPs, reaction conditions for a deblocking step may comprise the following: 700 mM NaNO₂; 1 M sodium acetate (adjusted with acetic acid to pH in the range of 4.8-6.5), where the deblocking reaction may be carried out in a 50 µL volume, at a temperature within the range of RT to 45°C for 30 seconds to several minutes.

Depending on particular applications, the steps of deblocking and/or cleaving may include a variety of chemical or physical conditions, e.g. light, heat, pH, presence of specific reagents, such as enzymes, which are able to cleave a specified chemical bond. Guidance in selecting 3'-O-blocking groups and corresponding de-blocking conditions may be found in the following references, which are incorporated by reference: U.S. patent 5808045; U.S. patent 8808988; International patent publication WO91/06678; and references cited below. In some embodiments, the cleaving agent (also sometimes referred to as a de-blocking reagent or agent) is a chemical cleaving agent, such as, for example, dithiothreitol (DTT). In alternative embodiments, a cleaving agent may be an enzymatic cleaving agent, such as, for example, a phosphatase, which may cleave a 3'-phosphate blocking group. It will be understood by the person skilled in the art that the selection of deblocking agent depends on the type of 3'-nucleotide blocking group used, whether one or multiple blocking groups are being used, whether initiators are attached to living cells or organisms or to solid supports, and the like, that necessitate mild treatment. For example, a phosphine, such as tris(2-carboxyethyl)phosphine (TCEP) can be used to cleave a 3'O-azidomethyl groups, palladium complexes can be used to cleave a 3'O-allyl groups, or sodium nitrite can be used to cleave a 3'O-amino group. In particular embodiments, the cleaving reaction involves TCEP, a palladium complex or sodium nitrite.

As noted above, in some embodiments it is desirable to employ two or more blocking groups that may be removed using orthogonal de-blocking conditions. The following exemplary pairs of blocking groups (Table 5) may be used in parallel synthesis embodiments, such as those described above. It is understood that other blocking group pairs, or groups containing more than two, may be available for use in these embodiments of the invention.

**Table 5: Exemplary pairs of blocking groups**

| | |
|---|---|
| 3'-O-NH2 | 3'-O-azidomethyl |
| 3'-O-NH2 | 3'-O-allyl |
| 3'-O-NH2 | 3'-O-phosphate |
| 3'-O-azidomethyl | 3'-O-allyl |
| 3'-O-azidomethyl | 3'-O-phosphate |
| 3'-O-allyl | 3'-O-phosphate |

Synthesizing oligonucleotides on living cells requires mild deblocking, or deprotection, conditions, that is, conditions that do not disrupt cellular membranes, denature proteins, interfere with key cellular functions, or the like. In some embodiments, deprotection conditions are within a range of physiological conditions compatible with cell survival. In such embodiments, enzymatic deprotection is desirable because it may be carried out under physiological conditions. In some embodiments specific enzymatically removable blocking groups are associated with specific enzymes for their removal. For example, ester- or acyl-based blocking groups may be removed with an esterase, such as acetylesterase, or like enzyme, and a phosphate blocking group may be removed with a 3' phosphatase, such as T4 polynucleotide kinase. By way of example, 3'-O-phosphates may be removed by treatment with as solution of 100 mM Tris-HCl (pH 6.5) 10 mM MgCl₂ , 5 mM 2-mercaptoethanol, and one Unit T4 polynucleotide kinase. The reaction proceeds for one minute at a temperature of 37°C.

A "3'-phosphate-blocked" or "3'-phosphate-protected" nucleotide refers to nucleotides in which the hydroxyl group at the 3'-position is blocked by the presence of a phosphate containing moiety. Examples of 3'-phosphate-blocked nucleotides in accordance with the invention arc nucleotidyl-3'-phosphate monoester/nucleotidyl-2',3'-cyclic phosphate, nuclcotidyl-2'-phosphate monoester and nucleotidyl-2' or 3'-alkylphosphate diester, and nucleotidyl-2' or 3'-pyrophosphate. Thiophosphate or other analogs of such compounds can also be used, provided that the substitution does not prevent dephosphorylation resulting in a free 3'-OH by a phosphatase.

Further examples of synthesis and enzymatic deprotection of 3'-O-ester-protected dNTPs or 3'-O-phosphate-protected dNTPs are described in the following references: Canard et al, Proc. Natl. Acad. Sci., 92:10859-10863 (1995); Canard et al, Gene, 148: 1-6 (1994); Cameron et al, Biochemistry, 16(23): 5120-5126 (1977); Rasolonjatovo et al, Nucleosides & Nucleotides, 18(4&5): 1021-1022 (1999); Ferrero et al, Monatshefte fur Chemie, 131: 585-616 (2000); Taunton-Rigby et al, J. Org. Chem., 38(5): 977-985 (1973); Uemura et al, Tetrahedron Lett., 30(29): 3819-3820 (1989); Becker et al, J. Biol. Chem., 242(5): 936-950 (1967); Tsien, International patent publication WO1991/006678.

As used herein, an "initiator" (or equivalent terms, such as, "initiating fragment," "initiator nucleic acid," "initiator oligonucleotide," or the like) refers to a short oligonucleotide sequence with a free 3'-end, which can be further elongated by a template-free polymerase, such as TdT. In one embodiment, the initiating fragment is a DNA initiating fragment. In an alternative embodiment, the initiating fragment is an RNA initiating fragment. In one embodiment, the initiating fragment possesses between 3 and 100 nucleotides, in particular between 3 and 20 nucleotides. In one embodiment, the initiating fragment is single-stranded. In an alternative embodiment, the initiating fragment is double-stranded. In a particular embodiment, an initiator oligonucleotide synthesized with a 5'-primary amine may be covalently linked to magnetic beads using the manufacturer's protocol. Likewise, an initiator oligonucleotide synthesized with a 3'-primary amine may be covalently linked to magnetic beads using the manufacturer's protocol. A variety of other attachment chemistries amenable for use with embodiments of the invention are well-known in the art, e.g. Integrated DNA Technologies brochure, "Strategies for Attaching Oligonucleotides to Solid Supports," v.6 (2014); Hermanson, Bioconjugate Techniques, Second Edition (Academic Press, 2008); and like references.

Many of the 3'-O-blocked dNTPs employed in the invention may be purchased from commercial vendors or synthesized using published techniques, e.g. U.S. patent 7057026; International patent publications WO2004/005667, WO91/06678; Canard et al, Gene (cited above); Metzker et al, Nucleic Acids Research, 22: 4259-4267 (1994); Meng et al, J. Org. Chem., 14: 3248-3252 (3006); U.S. patent publication 2005/037991. In some embodiments, the modified nucleotides comprise a modified nucleotide or nucleoside molecule comprising a purine or pyrimidine base and a ribose or deoxyribose sugar moiety having a removable 3'-OH blocking group covalently attached thereto, such that the 3' carbon atom has attached a group of the structure:

-O-Z

wherein -Z is any of -C(R')₂-O-R", -C(R')₂-N(R")₂, -C(R')₂-N(H)R", -C(R')₂-S-R" and - C(R')₂-F, wherein each R" is or is part of a removable protecting group; each R' is independently a hydrogen atom, an alkyl, substituted alkyl, arylalkyl, alkenyl, alkynyl, aryl, heteroaryl, heterocyclic, acyl, cyano, alkoxy, aryloxy, heteroaryloxy or amido group, or a detectable label attached through a linking group; with the proviso that in some embodiments such substituents have up to 10 carbon atoms and/or up to 5 oxygen or nitrogen heteroatoms; or (R')₂ represents a group of formula =C(R‴)₂ wherein each R‴ may be the same or different and is selected from the group comprising hydrogen and halogen atoms and alkyl groups, with the proviso that in some embodiments the alkyl of each R'" has from 1 to 3 carbon atoms; and wherein the molecule may be reacted to yield an intermediate in which each R" is exchanged for H or, where Z is -(R')₂-F, the F is exchanged for OH, SH or NH₂, preferably OH, which intermediate dissociates under aqueous conditions to afford a molecule with a free 3'-OH; with the proviso that where Z is -C(R')₂-S-R", both R' groups are not H. In certain embodiments, R' of the modified nucleotide or nucleoside is an alkyl or substituted alkyl, with the proviso that such alkyl or substituted alkyl has from 1 to 10 carbon atoms and from 0 to 4 oxygen or nitrogen heteroatoms. In certain embodiments, -Z of the modified nucleotide or nucleoside is of formula -C(R')₂-N3. In certain embodiments, Z is an azidomethyl group.

In some embodiments, Z is a cleavable organic moiety with or without heteroatoms having a molecular weight of 200 or less. In other embodiments, Z is a cleavable organic moiety with or without heteroatoms having a molecular weight of 100 or less. In other embodiments, Z is a cleavable organic moiety with or without heteroatoms having a molecular weight of 50 or less. In some embodiments, Z is an enzymatically cleavable organic moiety with or without heteroatoms having a molecular weight of 200 or less. In other embodiments, Z is an enzymatically cleavable organic moiety with or without heteroatoms having a molecular weight of 100 or less. In other embodiments, Z is an enzymatically cleavable organic moiety with or without heteroatoms having a molecular weight of 50 or less. In other embodiments, Z is an enzymatically cleavable ester group having a molecular weight of 200 or less. In other embodiments, Z is a phosphate group removable by a 3'-phosphatase. In some embodiments, one or more of the following 3'-phosphatases may be used with the manufacturer's recommended protocols: T4 polynucleotide kinase, calf intestinal alkaline phosphatase, recombinant shrimp alkaline phosphatase (e.g. available from New England Biolabs, Beverly, MA)

In further particular embodiments, the 3'-blocked nucleotide triphosphate is blocked by either a 3'-O-azidomethyl, 3'-O-NH₂ or 3'-O-allyl group.

In still other embodiments, 3'-O-blocking groups of the invention include 3'-O-methyl, 3'-O-(2-nitrobenzyl), 3'-O-allyl, 3'-O-amine, 3'-O-azidomethyl, 3'-O-tert-butoxy ethoxy, 3'-O-(2-cyanoethyl), and 3'-O-propargyl.

In some embodiments, 3'-O- protection groups are electrochemically labile groups. That is, deprotection or cleavage of the protection group is accomplished by changing the electrochemical conditions in the vicinity of the protection group which result in cleavage. Such changes in electrochemical conditions may be brought about by changing or applying a physical quantity, such as a voltage difference or light to activate auxiliary species which, in turn, cause changes in the electrochemical conditions at the site of the protection group, such as an increase or decrease in pH. In some embodiments, electrochemically labile groups include, for example, pH-sensitive protection groups that are cleaved whenever the pH is changed to a predetermined value. In other embodiments, electrochemically labile groups include protecting groups which are cleaved directly whenever reducing or oxidizing conditions are changed, for example, by increasing or decreasing a voltage difference at the site of the protection group.

A wide variety of protection groups (or equivalently, "base protecting moieties") may be employed to reduce or eliminate the formation of secondary structures in the course of polynucleotide chain extensions. Generally, the conditions for removing base protection groups are orthogonal to conditions for removing 3'-O-blocking groups. In particular, where removal, or de-blocking, conditions for 3'-O-blocking groups are acidic, then base protection groups may be selected to be base labile. Under such circumstances, many base labile protection groups have been developed in phosphoramidite synthesis chemistry due to the use of acid labile 5'-O-trityl-protected monomers, e.g. Beaucage and Iyer, Tetrahedron Letters, 48(12): 2223-2311 (1992). In particular, acyl and amidine protecting groups for phosphoramidite chemistry are applicable in embodiments of the present invention (e.g. the protecting groups of Table 2 and Table 3 of Beaucage and Iyer (cited above)). In some embodiments, base protecting groups are amidines, such as described in Table 2 of Beaucage and Iyer (cited above). Generally, base-protected 3'-O-blocked nucleoside triphosphate monomers may be synthesized by routine modifications of methods described in the literature, such as described in the examples below.

In some embodiments, a base protecting group is attached to the 6-nitrogen of deoxyadenosine triphosphate, the 2-nitrogen of deoxyguanosine triphosphate, and/or the 4-nitrogen of deoxycytidine triphosphate. In some embodiments, a base protecting group is attached to all of the indicated nitrogens. In some embodiments, a base protecting group attached to a 6-nitrogen of deoxyadenosine triphosphate is selected from the group consisting of benzoyl, phthaloyl, phenoxyacetyl, and methoxyacetyl; a base protecting group attached to the 2-nitrogen of deoxyguanosine triphosphate is selected from the group consisting of isobutyryl, isobutyryloxyethylene, acetyl, 4-isopropyl-phenoxyacetyl, phenoxyacetyl, and methoxyacetyl; and a base protecting group attached to said 4-nitrogen of deoxycytidine triphosphate is selected from the group consisting of benzoyl, phthaloyl, acetyl, and isobutyryl.

In some embodiments, a protecting group attached to the 6-nitrogen of deoxyadenosine triphosphate is benzoyl; a base protecting group attached to the 2-nitrogen of deoxyguanosine triphosphate is isobutryl or dimethylformamidine; and the base protecting group attached to the 4-nitrogen of deoxycytidine triphosphate is acetyl.

In some embodiments, a base protecting group attached to the 6-nitrogen of deoxyadenosine triphosphate is phenoxyacetyl; a base protecting group attached to the 2-nitrogen of deoxyguanosine triphosphate is 4-isopropyl-phenoxyacetyl or dimethylformamidine; and the base protecting group attached to the 4-nitrogen of deoxycytidine triphosphate is acetyl.

In some embodiments, base protecting moieties are removed (i.e. the product is deprotected) and product is cleaved from a solid support in the same reaction. For example, an initiator may comprise a ribo-uridine which may be cleaved to release the polynucleotide product by treatment with 1 M KOH, or like reagent (ammonia, ammonium hydroxide, NaOH, or the like), which simultaneously removes base-labile base protecting moieties.

When base-protected dNTPs are employed the above method may further include a step of removing base protecting moieties, which in the case of acyl or amidine protection groups may (for example) include treating with concentrated ammonia.

### Production of Variant TdTs

Variants of the invention may be produced by mutating known reference or wild type TdT-coding polynucleotides, then expressing it as a functional protein using conventional molecular biology techniques. For example, the mouse TdT gene encoding a truncated mouse TdT enzyme (SEQ ID NO: 2) may be assembled from synthetic fragments using conventional molecular biology techniques, e.g. using protocols described by Stemmer et al, Gene, 164: 49-53 (1995); Kodumal et al, Proc. Natl. Acad. Sci., 101: 15573-15578 (2004); or the like, or it may be directly cloned from mouse cells using protocols described by Boule et al, Mol. Biotechnology, 10: 199-208 (1998), or Bentolila et al, EMBO J., 14: 4221-4229 (1995); or the like.

For example, an isolated TdT gene may be inserted into an expression vector, such as pET32 (Novagen) to give a vector pCTdT which then may be used to make and express variant TdT proteins using conventional protocols. Vectors with the correct sequence may be transformed in E. coli producer strains.

Transformed strains are cultured using conventional techniques to pellets from which TdT protein is extracted. For example, previously prepared pellets are thawed in 30 to 37°C water bath. Once fully thawed, pellets are resuspended in lysis buffer composed of 50mM tris-HCL (Sigma) pH 7.5, 150mM NaCl (Sigma), 0.5mM mercaptoethanol (Sigma), 5% glycerol (Sigma), 20mM imidazole (Sigma) and 1 tab for 100mL of protease cocktail inhibitor (Thermofisher). Careful resuspension is carried out in order to avoid premature lysis and remaining of aggregates. Resuspended cells are lysed through several cycles of French press, until full color homogeneity is obtained. Usual pressure used is 14,000psi. Lysate is then centrifuged for 1h to 1h30 at 10,000 rpm. Centrifugate is pass through a 0.2µm filter to remove any debris before column purification.

TdT protein may be purified from the centrifugate in a one-step affinity procedure. For example, Ni-NTA affinity column (GE Healthcare) is used to bind the polymerases. Initially the column has been washed and equilibrated with 15 column volumes of 50mM tris-HCL (Sigma) pH 7.5, 150mM NaCl (Sigma) and 20mM imidazole (Sigma). Polymerases are bound to the column after equilibration. Then a washing buffer, composed of 50mM tris-HCL (Sigma) pH 7.5, 500mM NaCl (Sigma) and 20mM imidazole (Sigma), is applied to the column for 15 column volumes. After wash the polymerases are eluted with 50mM tris-HCL (Sigma) pH 7.5, 500mM NaCl (Sigma) and 0.5M imidazole (Sigma). Fractions corresponding to the highest concentration of polymerases of interest are collected and pooled in a single sample. The pooled fractions are dialyzed against the dialysis buffer (20 mM Tris-HCl, pH 6.8, 200mM Na Cl, 50mM MgOAc, 100mM [NH4]2SO4). The dialysate is subsequently concentrated with the help of concentration filters (Amicon Ultra-30, Merk Millipore). Concentrated enzyme is distributed in small aliquots, 50% glycerol final is added, and those aliquots are then frozen at -20°C and stored for long term. 5µL of various fraction of the purified enzymes are analyzed in SDSPAGE gels.

In some embodiments, a TdT variant may be operably linked to a linker moiety including a covalent or non-covalent bond; amino acid tag (e.g., poly-amino acid tag, poly-His tag, 6His-tag, or the like); chemical compound (e.g., polyethylene glycol); protein-protein binding pair (e.g., biotin-avidin); affinity coupling; capture probes; or any combination of these. The linker moiety can be separate from or part of a TdT variant. An exemplary His-tag for use with TdT variants of the invention is MASSHHHHHHSSGSENLYFQTGSSG- (SEQ ID NO: 19)). The tag-linker moiety does not interfere with the nucleotide binding activity, or catalytic activity of the TdT variant.

The above processes, or equivalent processes, result in an isolated TdT variant that may be mixed with a variety of reagents, such as, salts, pH buffers, carrier compounds, and the like, that are necessary or useful for activity and/or preservation.

### Kits for Practicing Methods of the Invention

The invention includes a variety of kits for practicing methods of the invention. In one aspect, kits of the invention comprise one or more chimeric TdT variants of the invention in a formulation, or in formulations, if provided separately, suitable for carrying out template-free enzymatic polynucleotide synthesis as described herein. Such kits may also include synthesis buffers for each TdT variant that provide reaction conditions for optimizing the template-free addition or incorporation of a 3'-O-protected dNTP to a growing strand. In some embodiments, each of the TdT variants may be provided separately, in the same or different formulations, in separate containers, and in other embodiments, some or all of the TdT variants may be provided in mixtures thereof in a common formulation. In some embodiments, kits of the invention further include 3'-O-reversibly protected dNTPs. In such embodiments, the 3'-O-reversibly protected dNTPs may comprise 3'-O-amino-dNTPs or 3'-O-azidomethyl-dNTPs. In further embodiments, kits may include one or more of the following items, either separately or together with the above-mentioned items: (i) deprotection or de-blocking reagents for carrying out a deprotecting or deblocking step as described herein, (ii) solid supports with initiators attached thereto, (iii) cleavage reagents for releasing completed polynucleotides from solid supports, (iv) wash reagents or buffers for removing unreacted 3'-O-reversibly protected dNTPs at the end of an enzymatic addition or coupling step, and (v) post-synthesis processing reagents, such as purification columns, desalting reagents, eluting reagents, and the like.

In regard to items (ii) and (iii) above, certain initiators and cleavage reagents go together. For example, an initiator comprising an inosine cleavable nucleotide may come with an endonuclease V cleavage reagent; an initiator comprising a nitrobenzyl photocleavable linker may come with a suitable light source for cleaving the photocleavable linker; an initiator comprising a uracil may come with a uracil DNA glycosylase cleavage reagent; and the like.

### Example 1: Chimeric Mouse-Bovine TdT Variants With Enhanced Activity For Incorporating 3'-O-Protected Deoxynucleoside Triphosphates

In this example, mouse-bovine chimeric TdT variants M57 (SEQ ID NO: 23) and M59 (SEQ ID NO: 24) constructed from mouse TdT variant M53 (SEQ ID NO: 21) and bovine TdT variant M54 (SEQ ID NO: 54) were tested for sequence-specific variability in dNTP incorporation by comparison to mouse TdT variant M27 (SEQ ID NO: 25). That is, M57 and M59 were tested for their efficiency of incorporating a 3'-O-blocked dNTP as a function of the three 3'-terminal nucleotides of a growing strand. For the test, twenty-four 52-mer random sequence polynucleotides were synthesized by each chimeric TdT variant (M57 and M59) along with mouse TdT variant M27 as a control. The syntheses were done in duplicate. The twenty-four 52-mers were selected so that each possible 4-mer was represented in the total (24x52 effective sequence) at approximately its expected frequency, i.e. 1/256, or about 4 or 5 occurences.

Synthesis conditions. Premixes containing two times concentrated dNTP-ONH2 (1 mM), DMSO (20%) and cofactor (4 mM CoCl₂) and two times concentrated mutants (32 µM) were prepared separately in TY 1x buffer 0.01% Tween20. For the M59 vs M27 reactions, the Tween20 was added 2x in the nucleotide premixes. The twenty-four sequences (52 mers) were synthesized in duplicates at 37°C with M27 and either M59 or M57. Elongation time was set at 4 min. The synthesis was performed using 500 pmole of dI resin (R133) and filter plates with wwPTFE membranes (Pall Laboratory, New York). TY1x buffer contains potassium cacodylate 200mM pH6.8, MgCl2 5mM. dI resin R133 is a CNBr sepharose resin (GE Healthcare) coupled with 10T-dI-T initial DNA (initiator DNA) via an amino function and linker is C12. So this is the complete sequence attached to the resin is 5AmMC12/TTTTTTTTTT/dI/T (SEQ ID NO: 83).

Post synthesis processing. After synthesis, first washes were performed (1x 100 µL/well H2O, 3x 100 µL/well SDS/DTT, 3x 100 µL/well H2O). For M57 vs M27 plates, no SDS/DTT washes were performed. Then, 3x 50 µL washes were performed with TH1 buffer 1x for M59 vs M27 plates or with WLE1 buffer 1x for M57 vs M27 plate immediately after removing the remaining liquid by vacuuming and centrifuging the plates 2 min at 2000 g. The cleavage to remove synthesized stands from the resin was performed directly after. For dI cleavage, resins were incubated with 1 µM in-house EndoV in 50 µL reaction volume (10 mM Tris pH7.9 containing 173 mM NaCl and 50 mM MgCl2 for M59 vs M27 plates or 10 mM Tris pH7.9 containing 50 mM KCl and 15 mM MgCl2 for M57 vs M27 plate) for 30 min, 42 °C, 1250 rpm then centrifuged at 2000 g for 2 min. Resins were washed with 25 µL TH1 buffer 1x for M59 vs M27 plates or with WLE1 buffer 1x for M57 vs M27 plate, incubated 2 more min at 42°C, 1250 rpm and centrifuged again at 2000 g for 2 min. The recovered filtrates were transferred to 96 well PCR plate and stored at -20°C if needed. At the end, less than 75 µL were collected from each well. TH1 buffer contains 10mM Tris, 170 mM NaCl, 50 MgCl2 pH8. WLE1 buffer contains 10 mM Tris pH7.9, 50 mM KCl and 15 mM MgCl2.

DNA precipitation. 0.5 µL glycogen and NaAc (3M) pH 5.2 (7.5 µL) were added per well and briefly shaked. 75 µL of isopropanol were added per well and centrifuged at 3428 g for 1h15 at 4°C. The supernatants were aspirated and only 20 µL were left in each well. 180 µL of freshly prepared ethanol 80% were added per well and centrifuged at 3428 g for 30 min at 4°C. The supernatants removal and the wash were repeated again. After centrifugation, only 10 µL per well were left to be dried in Speedvac at 35 °C for 30 min. The dried DNA were resuspended in 15 µL water MB grade, after which the concentrations were measured.

Library preparation: Libraries were prepared using the Accel-NGS 1S Plus DNA Library Kit for Illumina 1 96 rxns (ozyme SW10096). The libraries were indexed using the 1S Plus Indexing Kit for Illumina (ozyme SW16024).

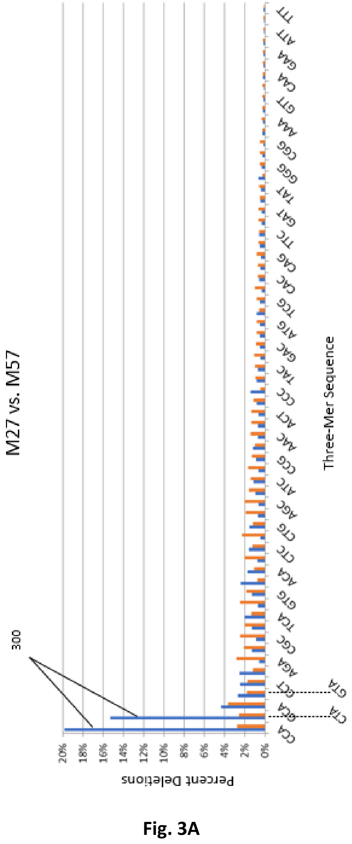

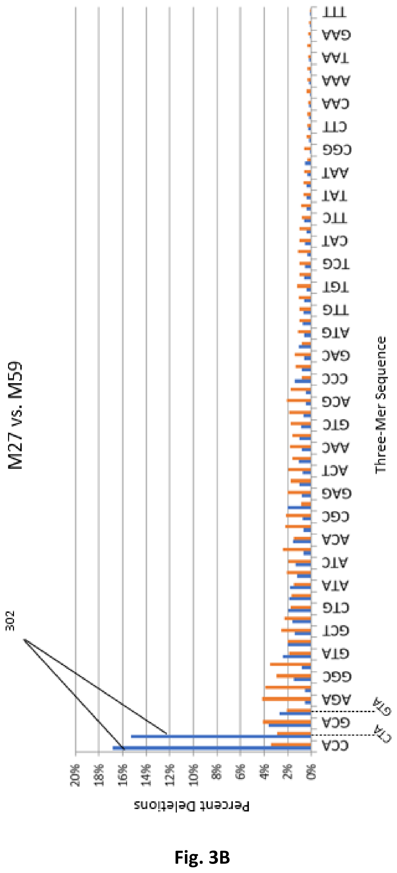

Figs. 3A and 3B show results derived from the sequencing data. In Fig. 3A, the frequencies (in percentages) of deletions occurring after different 3-mer sequences is compared for mouse TdT variant M27 (SEQ ID NO: 25) and mouse-bovine chimeric TdT variant M57 (SEQ ID NO: 23). In Fig. 3B, the frequencies of deletions occurring after different 3-mer sequences is compared for mouse TdT variant M27 (SEQ ID NO: 25) and mouse-bovine chimeric TdT variant M59 (SEQ ID NO: 24). Note that, for the sake of legibility, except for the first four 3-mers, only every other pair of data bars are labeled with its corresponding 3-mer. The important difference between prior TdT variants (as exemplified by M27) and chimeric TdT variants (as exemplified by M57 and M59) is the minimization of sequence-specific insertion of deletions by chimeric TdT variants M57 and M59, especially for occurrences of the triplets, CCA and CTA (300 and 302 in Figs. 3A and 3B, respectively), in the polynucleotide being synthesized. For both of these triplets, the rate of deletions after the triplets for either M57 or M59 is about 25 percent or less than the rate from M27.

### Definitions

Amino acids are represented by either their one-letter or three-letters code according to the following nomenclature: A: alanine (Ala); C: cysteine (Cys); D: aspartic acid (Asp); E: glutamic acid (Glu); F: phenylalanine (Phe); G: glycine (Gly); H: histidine (His); I: isoleucine (Ile); K: lysine (Lys); L: leucine (Leu); M: methionine (Met); N: asparagine (Asn); P: proline (Pro); Q: glutamine (Gln); R: arginine (Arg); S: serine (Ser); T: threonine (Thr); V: valine (Val); W: tryptophan (Trp ) and Y: tyrosine (Tyr).

"Functionally equivalent" in reference to amino acid positions in two or more different TdTs means (i) the amino acids at the respective positions play the same functional role in an activity of the TdTs, and (ii) the amino acids occur at homologous amino acid positions in the amino acid sequences of the respective TdTs. It is possible to identify positionally equivalent or homologous amino acid residues in the amino acid sequences of two or more different TdTs on the basis of sequence alignment and/or molecular modelling. In some embodiments, functionally equivalent amino acid positions belong to sequence motifs that are conserved among the amino acid sequences of TdTs of evolutionarily related species, e.g. related by genus, families, or the like. Examples of such conserved sequence motifs are described in Motea et al, Biochim. Biophys. Acta. 1804(5): 1151-1166 (2010); Delarue et al, EMBO J., 21: 427-439 (2002); and like references.

"Isolated" in reference to protein means such a compound which has been identified and separated and/or recovered from a component of its natural environment or from a heterogeneous reaction mixture. Contaminant components of a natural environment or reaction mixture are materials which would interfere with a protein's function, and may include enzymes, hormones, and other proteinaceous or nonproteinaceous solutes. In some embodiments, a protein of the invention is purified (1) to greater than 95% by weight of protein as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. When manufactured by recombinant methodologies, an isolated protein of the invention may include the protein of the invention in situ within recombinant cells since at least one component of the protein's natural environment will not be present. Ordinarily, an isolated protein of the invention is prepared by at least one purification step.

"Kit" refers to any delivery system for delivering materials or reagents for carrying out a method of the invention. In the context of reaction assays, such delivery systems include systems and/or compounds (such as dilutants, surfactants, carriers, or the like) that allow for the storage, transport, or delivery of reaction reagents (e.g., one or more TdT variants, reaction buffers, 3'-O-protected-dNTPs, deprotection reagents, solid suppprts with initiators attached , etc. in the appropriate containers) and/or supporting materials (e.g., buffers, written instructions for performing the assay etc.) from one location to another. For example, kits include one or more enclosures (e.g., boxes) containing the relevant reaction reagents and/or supporting materials. Such contents may be delivered to the intended recipient together or separately. For example, a first container may contain one or more TdT variants for use in a synthesis method, while a second or additional containers may contain deprotection agents, solid supports with initiators, 3'-O-protected dNTPs, or the like.

"Mutant" or "variant," which are used interchangeably, refer to a polypeptide derived from a specified (usually wild type or natural) amino acid sequence, such as, SEQ ID NO: 2, which contains a modification or an alteration, e.g., a substitution, insertion, and/or deletion, relative to the specified sequence at one or more positions. Such mutants or variants usually have both a template-free polymerase activity and ability to incorporate one or more reversibly blocked nucleoside triphosphate precursors. The variants may be obtained by various techniques well known in the art. In particular, examples of techniques for altering the DNA sequence encoding a wild-type protein, include, but are not limited to, site-directed mutagenesis, random mutagenesis and synthetic oligonucleotide construction, and the like. Mutagenesis activities consist in deleting, inserting or substituting one or several amino-acids in the sequence of a protein or in the case of the invention of a polymerase.

"Sequence identity" refers to the number (or fraction, usually expressed as a percentage) of matches (e.g., identical amino acid residues) between two sequences, such as two polypeptide sequences or two polynucleotide sequences. The sequence identity is determined by comparing the sequences when aligned so as to maximize overlap and identity while minimizing sequence gaps. In particular, sequence identity may be determined using any of a number of mathematical global or local alignment algorithms, depending on the length of the two sequences. Sequences of similar lengths are preferably aligned using a global alignment algorithm (e.g. Needleman and Wunsch algorithm; Needleman and Wunsch, 1970) which aligns the sequences optimally over the entire length, while sequences of substantially different lengths are preferably aligned using a local alignment algorithm (e.g. Smith and Waterman algorithm (Smith and Waterman, 1981) or Altschul algorithm (Altschul et al., 1997; Altschul et al., 2005)). Alignment for purposes of determining percent amino acid sequence identity can be achieved in various ways that are within the skill in the art, for instance, using publicly available computer software available on internet web sites such as http://blast.ncbi.nlm.nih.gov/ or ttp://www.ebi.ac.uk/Tools/emboss/. Those skilled in the art can determine appropriate parameters for measuring alignment, including any algorithm needed to achieve maximal alignment over the full length of the sequences being compared. For purposes herein, % amino acid sequence identity values refer to values generated using the pair wise sequence alignment program EMBOSS Needle, that creates an optimal global alignment of two sequences using the Needleman-Wunsch algorithm, wherein all search parameters are set to default values, i.e. Scoring matrix = BLOSUM62, Gap open = 10, Gap extend = 0.5, End gap penalty = false, End gap open = 10 and End gap extend = 0.5.

"Polynucleotide" or "oligonucleotide" are used interchangeably and each mean a linear polymer of nucleotide monomers or analogs thereof. Monomers making up polynucleotides and oligonucleotides are capable of specifically binding to a natural polynucleotide by way of a regular pattern of monomer-to-monomer interactions, such as Watson-Crick type of base pairing, base stacking, Hoogsteen or reverse Hoogsteen types of base pairing, or the like. Such monomers and their internucleosidic linkages may be naturally occurring or may be analogs thereof, e.g. naturally occurring or non-naturally occurring analogs. Non-naturally occurring analogs may include PNAs, phosphorothioate internucleosidic linkages, bases containing linking groups permitting the attachment of labels, such as fluorophores, or haptens, and the like. Whenever the use of an oligonucleotide or polynucleotide requires enzymatic processing, such as extension by a polymerase, ligation by a ligase, or the like, one of ordinary skill would understand that oligonucleotides or polynucleotides in those instances would not contain certain analogs of internucleosidic linkages, sugar moieties, or bases at any or some positions. Polynucleotides typically range in size from a few monomeric units, e.g. 5-40, when they are usually referred to as "oligonucleotides," to several thousand monomeric units. Whenever a polynucleotide or oligonucleotide is represented by a sequence of letters (upper or lower case), such as "ATGCCTG," it will be understood that the nucleotides are in 5'-3' order from left to right and that "A" denotes deoxyadenosine, "C" denotes deoxycytidine, "G" denotes deoxyguanosine, and "T" denotes thymidine, "I" denotes deoxyinosine, "U" denotes uridine, unless otherwise indicated or obvious from context. Unless otherwise noted the terminology and atom numbering conventions will follow those disclosed in Strachan and Read, Human Molecular Genetics 2 (Wiley-Liss, New York, 1999). Usually polynucleotides comprise the four natural nucleosides (e.g. deoxyadenosine, deoxycytidine, deoxyguanosine, deoxythymidine for DNA or their ribose counterparts for RNA) linked by phosphodiester linkages; however, they may also comprise non-natural nucleotide analogs, e.g. including modified bases, sugars, or internucleosidic linkages. It is clear to those skilled in the art that where an enzyme has specific oligonucleotide or polynucleotide substrate requirements for activity, e.g. single stranded DNA, RNA/DNA duplex, or the like, then selection of appropriate composition for the oligonucleotide or polynucleotide substrates is well within the knowledge of one of ordinary skill, especially with guidance from treatises, such as Sambrook et al, Molecular Cloning, Second Edition (Cold Spring Harbor Laboratory, New York, 1989), and like references. Likewise, the oligonucleotide and polynucleotide may refer to either a single stranded form or a double stranded form (i.e. duplexes of an oligonucleotide or polynucleotide and its respective complement). It will be clear to one of ordinary skill which form or whether both forms are intended from the context of the terms usage.

"Primer" means an oligonucleotide, either natural or synthetic that is capable, upon forming a duplex with a polynucleotide template, of acting as a point of initiation of nucleic acid synthesis and being extended from its 3' end along the template so that an extended duplex is formed. Extension of a primer is usually carried out with a nucleic acid polymerase, such as a DNA or RNA polymerase. The sequence of nucleotides added in the extension process is determined by the sequence of the template polynucleotide. Usually primers are extended by a DNA polymerase. Primers usually have a length in the range of from 14 to 40 nucleotides, or in the range of from 18 to 36 nucleotides. Primers are employed in a variety of nucleic amplification reactions, for example, linear amplification reactions using a single primer, or polymerase chain reactions, employing two or more primers. Guidance for selecting the lengths and sequences of primers for particular applications is well known to those of ordinary skill in the art, as evidenced by the following references that are incorporated by reference: Dieffenbach, editor, PCR Primer: A Laboratory Manual, 2nd Edition (Cold Spring Harbor Press, New York, 2003).

A "substitution" means that an amino acid residue is replaced by another amino acid residue. Preferably, the term *"substitution"* refers to the replacement of an amino acid residue by another selected from the naturally-occurring standard 20 amino acid residues, rare naturally occurring amino acid residues (e.g. hydroxyproline, hydroxylysine, allohydroxylysine, 6-N-methylysine, N-ethylglycine, N-methylglycine, N-ethylasparagine, allo-isoleucine, N-methylisoleucine, N-methylvaline, pyroglutamine, aminobutyric acid, ornithine, norleucine, norvaline), and non-naturally occurring amino acid residue, often made synthetically, (e.g. cyclohexyl-alanine). Preferably, the term *"substitution"* refers to the replacement of an amino acid residue by another selected from the naturally-occurring standard 20 amino acid residues. The sign "+" indicates a combination of substitutions.

The amino acids are herein represented by their one-letter or three-letters code according to the following nomenclature: A: alanine (Ala); C: cysteine (Cys); D: aspartic acid (Asp); E: glutamic acid (Glu); F: phenylalanine (Phe); G: glycine (Gly); H: histidine (His); I: isoleucine (Ile); K: lysine (Lys); L: leucine (Leu); M: methionine (Met); N: asparagine (Asn); P: proline (Pro); Q: glutamine (Gln); R: arginine (Arg); S: serine (Ser); T: threonine (Thr); V: valine (Val); W: tryptophan (Trp ) and Y: tyrosine (Tyr).

In the present document, the following terminology is used to designate a substitution: L238A denotes that amino acid residue (Leucine, L) at position 238 of the parent sequence is changed to an Alanine (A). A132V/I/M denotes that amino acid residue (Alanine, A) at position 132 of the parent sequence is substituted by one of the following amino acids: Valine (V), Isoleucine (I), or Methionine (M). The substitution can be a conservative or non-conservative substitution. Examples of conservative substitutions are within the groups of basic amino acids (arginine, lysine and histidine), acidic amino acids (glutamic acid and aspartic acid), polar amino acids (glutamine, asparagine and threonine), hydrophobic amino acids (methionine, leucine, isoleucine, cysteine and valine), aromatic amino acids (phenylalanine, tryptophan and tyrosine), and small amino acids (glycine, alanine and serine).

"Wild type" in reference to the amino acid sequence of a protein means an amino acid sequence found in nature.

"Motif" as used herein in reference to amino acid sequences of TdT variants means a subsequence or segment of a TdT amino acid sequence at a particular location which is conserved (but may not be identical) among the amino acid sequences of TdTs of different species, especially of evolutionarily related species. Examples of such conserved sequence motifs are described in Motea et al, Biochim. Biophys. Acta. 1804(5): 1151-1166 (2010); Delarue et al, EMBO J., 21: 427-439 (2002); and like references. Exemplary TdT motifs include (ordered from N-terminus to C-terminus): FMR, VSC, GGFRR, KMT, GHD, TGSR, FER and the like. As referenced herein, a sequence motif is identified by the most common sequence occurrence or a consensus sequence, e.g. "TGSR", although such designations are not meant to exclude other subsequences that align with TGSR using a conventional alignment tool, such as Multalin (Mitchell, Bioinformatics, 9(5): 614-615 (1993)). For example, reference to the "TGSR motif" includes the segment "SGSR" of possum TdT which aligns with the segment "TGSR" of mouse. In some embodiments, a sequence motif is defined as an express set of sequences. For example, in some embodiments, the FMR motif consists of the sequences in the group consisting of FMR, FLR and YMR.

This disclosure is not intended to be limited to the scope of the particular forms set forth, but is intended to cover alternatives, modifications, and equivalents of the variations described herein. Further, the scope of the disclosure fully encompasses other variations that may become obvious to those skilled in the art in view of this disclosure. The scope of the present invention is limited only by the appended claims.
The present application discloses the following items:
1. A chimeric terminal deoxynucleotidyl transferase (TdT) variant comprising an amino acid sequence at least 80 percent identical to the amino acid sequence defined by the formula:

   B₁-B₂-B₃

   wherein:
   B₁ is an amino acid segment of a first species TdT without a BRCT-like region, extending from its N-terminus to an amino acid position between its VSC motif and its KMT motif inclusive, wherein B₁ comprises a substitution of methionine or functionally equivalent residue in its FMR motif, a substitution of the second arginine or functionally equivalent residue in its GGFRR motif whenever B₁ includes a GGFRR motif, and one or more substitutions of valine or serine or cysteine, or functionally equivalent residue, respectively, in its VSC motif whenever B₁ includes a VSC motif;
   B₂ is an amino acid segment of a second species TdT extending from a C-terminus of B₁ to a C-terminus of the second species TdT, or to the N-terminus of B₃ whenever B₃ comprises one or more amino acids, wherein B₂ comprises one or more substitutions of valine or serine or cysteine, or functionally equivalent residue, respectively, in its VSC motif whenever B₂ includes a VSC motif, a substitution of the second arginine or functionally equivalent residue in its GGFRR motif whenever B₂ includes a GGFRR motif, a substitution of arginine or functionally equivalent residue in its TGSR motif whenever B₂ includes a TGSR motif, and a substitution of glutamic acid or functionally equivalent residue in its FER motif whenever B₂ includes a FER motif; and
   B₃ is an amino acid segment of a third species TdT comprising from 0 to 70 amino acids and extending from a C-terminus of the third species TdT to a C-terminal amino acid of B₂, wherein B₃ comprises a substitution of arginine or functionally equivalent residue in its TGSR motif whenever B₃ includes a TGSR motif, and a substitution of glutamic acid or functionally equivalent residue in its FER motif whenever B₃ includes a FER motif;
   and wherein the chimeric TdT variant (i) is capable of synthesizing a nucleic acid fragment without a template and (ii) is capable of incorporating a 3'-O-blocked nucleoside triphosphate onto a nucleic acid fragment;
   and wherein B₁, B₂ and B₃ are selected so that the chimeric TdT variant has from 350 to 410 amino acids.
2. The chimeric TdT variant of item 1 wherein:
   (i) said substitution of methionine or functionally equivalent residue in said FMR motif of B₁ is R or Q; said substitution of said second arginine or functionally equivalent residue in said GGFRR motif of B₁ whenever B₁ includes a GGFRR motif is L or N; and said substitution of cysteine or functionally equivalent residue, in said VSC motif of B₁ whenever B₁ includes a VSC motif is G or R;
   (ii) said substitution of cysteine or functionally equivalent residue in said VSC motif of B₂ whenever B₂ includes a VSC motif is R or Q; said substitution of said second arginine or functionally equivalent residue in said GGFRR motif of B₂ whenever B₂ includes a GGFRR motif is L or N; said substitution of arginine or functionally equivalent residue in said TGSR motif of B₂ whenever B₂ includes a TGSR motif is P, N or A; and said substitution of glutamic acid or functionally equivalent residue in said FER motif of B₂ whenever B₂ includes a FER motif is N, L, T, or S; and
   (iii) said substitution of arginine or functionally equivalent residue in said TGSR motif of B₃ whenever B₃ includes a TGSR motif is P, N or A; and said substitution of glutamic acid or functionally equivalent residue in said FER motif of B₃ whenever B₃ includes a FER motif is N, L, T or S.
3. The chimeric TdT variant of item 1 or 2, wherein said first species TdT and said third species TdT are the same.
4. The chimeric TdT variant of any one of item 1 to 3 wherein each of said amino acid segments B₁, B₂ and B₃ has an amino acid sequence at least 90 percent identical to an amino acid segment of a sequence selected from the group consisting of SEQ ID NOs 2, 3, 4, 5, 6, 7, 8, 9, 10, 11, 12, 13, 14, 15, 26, 27, 28, 29, 30, 31, 32, 33, 34, 35, 36, 37, 38, 39, 40 and 41 and wherein:
   said substitution of said methionine or its functionally equivalent residue in said FMR motif is at position 63 with respect to SEQ ID NOs: 2, 3, 4, 6, 7, 8, 11, 12 and 13; at position 62 with respect to SEQ ID NO: 5; position 64 with respect to SEQ ID NO: 9; position 61 with respect to SEQ ID NO: 10, 26, 28, 29, 30, 32, 33, 34, 36, 37, 38 and 39; position 66 with respect to SEQ ID NO: 14; position 47 with respect to SEQ ID NO: 15; and position 48 with respect to SEQ ID NO: 27, 31, 35 and 40;
   said substitution of said valine or its functionally equivalent residue in said VSC motif is at position 171 with respect to SEQ ID NOs: 2, 3, 4, 6, 7, 8, 11 and, 12; position 170 with respect to SEQ ID NO: 5, 32, 33, 34, 36, 37, 38, 39 and 41; position 172 with respect to SEQ ID NO: 9; position 169 with respect to SEQ ID NO: 10, 28, 29, 30, 32, 33, 34, 36, 37, 38, 38 and 41; position 180 with respect to SEQ ID NO: 13; position 174 with respect to SEQ ID NO: 14; position 154 with respect to SEQ ID NO: 15; position 168 with respect to SEQ ID NO: 26; position 156 with respect to SEQ ID NO: 27, 35 and 40; position 157 with respect to SEQ ID NO: 31;;
   said substitution of said serine or its functionally equivalent residue in said VSC motif is at position 172 with respect to SEQ ID NOs: 2, 3, 4, 6, 7, 8, 11 and 12; position 171 with respect to SEQ ID NO: 5; position 173 with respect to SEQ ID NO: 9; position 170 with respect to SEQ ID NO: 10, 28, 29, 30; position 181 with respect to SEQ ID NO: 13; position 175 with respect to SEQ ID NO: 14; position 155 with respect to SEQ ID NO: 15; position 169 with respect to SEQ ID NO: 26 and position 157 with respect to SEQ ID NO: 27, 31, 35 and 40;
   said substitution of said cysteine or its functionally equivalent residue in said VSC motif is at position 173 with respect to SEQ ID NOs: 2, 3, 4, 6, 7, 8, 11 and 12; position 172 with respect to SEQ ID NO: 5; position 174 with respect to SEQ ID NO: 9; position 171 with respect to SEQ ID NO: 10, 28, 29, 30, 32, 33, 34, 37, 38, 39 and 41; position 182 with respect to SEQ ID NO: 13; position 176 with respect to SEQ ID NO: 14; position 170 with respect to SEQ ID NO: 26; position 158 with respect to SEQ ID NO: 27, 31, 35, 36 and 40; and position 156 with respect to SEQ ID NO: 15;
   said substitution of said second arginine or its functionally equivalent residue in said GGFRR motif is at position 207 with respect to SEQ ID NOs: 2, 3, 4, 6, 7, 8, 11 and 12; position 206 with respect to SEQ ID NO: 5; position 208 with respect to SEQ ID NO: 9; position 205 with respect to SEQ ID NO: 10, 28, 29, 30, 32, 33, 34, 36, 37, 38, 39 and 41; position 204 with respect to SEQ ID NO: 26; position 192 with respect to SEQ ID NO: 27, 31, 35 and 40; position 216 with respect to SEQ ID NO: 13; position 210 with respect to SEQ ID NO: 14; and position 190 with respect to SEQ ID NO: 15;
   said substitution of said arginine or functionally equivalent residue in said TGSR motif is at position 325 with respect to SEQ ID NOs: 2, 7, 8, 12 and 33; position 324 with respect to SEQ ID NOs: 3, 4, 29 and 32; position 320 with respect to SEQ ID NO: 5; position 331 with respect to SEQ ID NO: 6; position 326 with respect to SEQ ID NO: 9 and 26; position 327 with respect to SEQ ID NO: 28, 30; position 323 with respect to SEQ ID NO: 10; position 328 with respect to SEQ ID NO: 11 and 14; position 338 with respect to SEQ ID NO: 13; position 314 with respect to SEQ ID NO: 27, 31; position 310 with respect to SEQ ID NO: 35; position 311 with respect to SEQ ID NO: 31; position 321 with respect to SEQ ID NO: 32; position 322 with respect to SEQ ID NO: 33, 34; position 323 with respect to SEQ ID NO: 36, 37, 38, 39 and 41; position 309 with respect to SEQ ID NO: 40; and position 308 with respect to SEQ ID NO: 15; and
   said substitution of said glutamic acid or functionally equivalent residue in said FER motif is at position 328 with respect to SEQ ID NO: 2, 7, 8 and 12; position 327 with respect to SEQ ID NO: 3, 4 and 29; position 323 with respect to SEQ ID NO: 5; position 334 with respect to SEQ ID NO: 6; position 329 with respect to SEQ ID NO: 9 and 26; position 326 with respect to SEQ ID NO: 10, 36, 37, 38, 39 and 41; position 331 with respect to SEQ ID NO: 11 and 14; position 341 with respect to SEQ ID NO: 13; position 317 with respect to SEQ ID NO: 27; position 330 with respect to SEQ ID NO: 28 and 30; position 312 with respect to SEQ ID NO: 40; position 313 with respect to SEQ ID NO: 35; position 314 with respect to SEQ ID NO: 31; position 324 with respect to SEQ ID NO: 32; position 325 with respect to SEQ ID NO: 33 and 34; and position 311 with respect to SEQ ID NO: 15.
5. The chimeric TdT variant of item 4 wherein:
   (i) said substitution of methionine or functionally equivalent residue in said FMR motif of B₁ is R or Q; said substitution of said second arginine or functionally equivalent residue in said GGFRR motif of B₁ whenever B₁ includes a GGFRR motif is L or N; and said substitution of cysteine or functionally equivalent residue, in said VSC motif of B₁ whenever B₁ includes a VSC motif is G or R;
   (ii) said substitution of cysteine or functionally equivalent residue in said VSC motif of B₂ whenever B₂ includes a VSC motif is R or Q; said substitution of said second arginine or functionally equivalent residue in said GGFRR motif of B₂ whenever B₂ includes a GGFRR motif is L or N; said substitution of arginine or functionally equivalent residue in said TGSR motif of B₂ whenever B₂ includes a TGSR motif is P, N or A; and said substitution of glutamic acid or functionally equivalent residue in said FER motif of B₂ whenever B₂ includes a FER motif is N, L, T, or S; and
   (iii) said substitution of arginine or functionally equivalent residue in said TGSR motif of B₃ whenever B₃ includes a TGSR motif is P, N or A; and said substitution of glutamic acid or functionally equivalent residue in said FER motif of B₃ whenever B₃ includes a FER motif is N, L, T or S.
6. The chimeric TdT variant of item 4 wherein said substitution of said methionine of said FMR motif is R or Q; said substitution of said valine of said VSC motif is A; said substitution of said serine of said VSC motif is E; said substitution of said cysteine of said VSC motif is G or R; said substitution of said second arginine of said GGFRR motif is L or N; said substitution of said arginine of said TGSR motif is P, N or A; and said substitution of said glutamic acid of said FER motif is N, L, T or S.
7. The chimeric TdT variant of any of item 1-6 wherein said first and second species TdTs are each mammalian TdTs.
8. The chimeric TdT variant of item 7 wherein said first species TdT and said second species TdT are each TdTs of a species selected from group consisting of mouse, bovine, human, possum, elephant shrew, canine, mole, pika, hedgehog, tree shrew, platypus, jerboa and puma.
9. The chimeric TdT variant of item 8 wherein said first species TdT is mouse and said second species TdT is bovine.
10. The chimeric TdT variant of item 9 wherein said first species TdT has an amino acid sequence of SEQ ID NO: 21 and said second species TdT has an amino acid sequence of SEQ ID NO: 22.
11. The chimeric TdT variant of item 10 having an amino acid sequence of SEQ ID NO: 23.
12. The chimeric TdT variant of item 10 having an amino acid sequence of SEQ ID NO: 24.
13. The chimeric TdT variant of any of items 1 to 12, wherein said 3'-O-modified nucleoside triphosphate is a 3'-O-NH₂-nucleoside triphosphate, a 3'-O-azidomethyl-nucleoside triphosphate, a 3'-O-allyl-nucleoside triphosphate, or a 3'-O-(2-nitrobenzyl)-nucleoside triphosphate.
14. A chimeric terminal deoxynucleotidyl transferase (TdT) variant comprising an amino acid sequence at least 80 percent identical to an amino acid sequence defined by the formula:

   J₁-J₂-J₃

   wherein:
   J₁ is an amino acid segment of a mouse TdT without a BRCT-like region extending from its N-terminus to an amino acid position between a VSC motif and a KMT motif, inclusive, wherein J₁ comprises a substitution of methionine or functionally equivalent residue in a FMR motif, a substitution of the second arginine or functionally equivalent residue in its GGFRR motif whenever J₁ includes a GGFRR motif, and one or more substitutions of valine or serine or cysteine, or functionally equivalent residue, respectively, in the VSC motif whenever J₁ includes a VSC motif;
   J₂ is an amino acid segment of a non-mouse TdT extending from an amino acid position located between a VSC motif and a KMT motif to an amino acid position at a TGSR motif, wherein the amino acid segment comprises a substitution of the second arginine or functionally equivalent residue in its GGFRR motif whenever J₂ includes a GGFRR motif, and one or more substitutions of valine or serine or cysteine, or functionally equivalent residue, respectively, in its VSC motif whenever J₂ includes a VSC motif; and
   J₃ is an amino acid segment of a mouse TdT extending from an amino acid position at a TGSR motif to its C-terminal amino acid, wherein J₃ comprises a substitution of arginine or functionally equivalent residue in its TGSR motif whenever J₃ includes a TGSR motif, and a substitution of glutamic acid or functionally equivalent residue in its FER motif whenever J₃ includes a FER motif;
   and wherein the chimeric TdT variant (i) is capable of synthesizing a nucleic acid fragment without a template and (ii) is capable of incorporating a 3'-O-blocked nucleoside triphosphate onto a nucleic acid fragment;
   and wherein J₁, J₂ and J₃ are selected so that the chimeric TdT variant has from 350 to 410 amino acids.
15. The chimeric TdT variant of item 14 wherein (i) said amino acid segment J₁ comprises an amino acid sequence selected from SEQ ID NO: 42, 71, 73 or 82; and the amino acid sequence of SEQ ID NO: 42 has a substitution of methionine at position 63, (ii) said amino acid segment J₂ comprises an amino acid sequence selected from the group consisting of SEQ ID NOs: 44, 45, 46, 47, 48, 49, 50, 51, 52, 53, 54, 55, 56, 57, 58, 59, 60, 61, 62, 63, 64, 65, 66, 67, 68, 69, an 70, each with a substitution of arginine at position 35, and each having a substitution of cysteine at position 1, and (iii) said amino acid segment J₃ comprises an amino acid sequence of SEQ ID NO: 43 with a substitution of arginine at position 12, glutamic acid at position 15, and a substitution of arginine at position 38; or an amino acid sequence of SEQ ID NO: 72.
16. The chimeric TdT variant of item 15 wherein said substitution of methionine at position 63 is R or Q, wherein said substitution of arginine at position 35 is L or N, and wherein said substitution of cysteine at positions 1 is G or R.
17. The chimeric TdT variant of item 15 or 16, wherein said amino acid sequence of SEQ ID NO: 42 further has substitutions of alanine at position 17, leucine at position 52, glycine at position 57, methionine at position 63 and alanine at position 108.
18. The chimeric TdT variant of any one of items 14 to 17, wherein said non-mouse TdT is bovine TdT.
19. The chimeric TdT variant of items 18, wherein said chimeric TdT variant has an amino acid sequence selected from the group consisting of SEQ ID NO: 74, 75, 76, 77, 78, 79, 80 and 81.
20. The chimeric TdT variant of any of items 14 to 19, wherein said 3'-O-modified nucleoside triphosphate is a 3'-O-NH2-nucleoside triphosphate, a 3'-O-azidomethyl-nucleoside triphosphate, a 3'-O-allyl-nucleoside triphosphate, or a 3'-O-(2-nitrobenzyl)-nucleoside triphosphate.
21. A method of synthesizing a polynucleotide having a predetermined sequence, the method comprising the steps of:
   a) providing an initiator having a free 3'-hydroxyl; and
   b) repeating cycles of (i) contacting under elongation conditions the initiator or elongated fragments having free 3'-O-hydroxyls with a 3'-O-blocked nucleoside triphosphate and a terminal deoxynucleotidyltransferase (TdT) variant so that the initiator or elongated fragments are elongated by incorporation of a 3'-O-blocked nucleoside triphosphate to form 3'-O-blocked elongated fragments, and (ii) deblocking the elongated fragments to form elongated fragments having free 3'-hydroxyls, until the polynucleotide is synthesized, wherein the TdT variant a chimeric TdT variant of any of items 1 to 20.
22. A kit for performing a nucleotide incorporation reaction comprising: a) a chimeric TdT variant according to any one of items 1 to 20, b) one or more nucleotides, preferably one or more 3'-O-blocked nucleosides triphosphates, and c) optionally at least one nucleic acid primer.

## Claims

1. A chimeric terminal deoxynucleotidyl transferase (TdT) variant comprising an amino acid sequence at least 90 percent identical to an amino acid sequence defined by the formula:
J₁-J₂-J₃
wherein:
J₁ is an amino acid segment having an amino acid sequence SEQ ID NO: 73;
J₂ is an amino acid segment having an amino acid sequence of SEQ ID NO: 58 with a substitution of arginine at position 35; and
J₃ is an amino acid segment having an amino acid sequence SEQ ID NO:72,
and wherein the chimeric TdT variant (i) is capable of synthesizing a nucleic acid fragment without a template and (ii) is capable of incorporating a 3'-O-blocked nucleoside triphosphate onto a nucleic acid fragment.

2. The chimeric TdT variant of claim 1, comprising an amino acid sequence SEQ ID NO:24.

3. The chimeric TdT variant of any of claims 1 or 2, wherein said 3'-O-blocked nucleoside triphosphate is a 3'-O-NH₂-nucleoside triphosphate, a 3'-O-azidomethyl-nucleoside triphosphate, a 3'-O-allyl-nucleoside triphosphate, or a 3'-O-(2-nitrobenzyl)-nucleoside triphosphate.

4. A method of synthesizing a polynucleotide having a predetermined sequence, the method comprising the steps of:
a) providing an initiator having a free 3'-hydroxyl; and
b) repeating cycles of (i) contacting under elongation conditions the initiator or elongated fragments having free 3'-O-hydroxyls with a 3'-O-blocked nucleoside triphosphate and a terminal deoxynucleotidyl transferase (TdT) variant so that the initiator or elongated fragments are elongated by incorporation of a 3'-O-blocked nucleoside triphosphate to form 3'-O-blocked elongated fragments, and (ii) deblocking the elongated fragments to form elongated fragments having free 3'-hydroxyls, until the polynucleotide is synthesized, wherein the TdT variant is a chimeric TdT variant of any of claims 1 to 3.

5. A kit for performing a nucleotide incorporation reaction comprising: a) a chimeric TdT variant according to any one of claims 1 to 3, b) one or more nucleotides, preferably one or more 3'-O-blocked nucleosides triphosphates, and c) optionally at least one initiator having a free 3'-hydroxyl.
